# EUROPEAN PATENT SPECIFICATION

(11) **EP 2 019 834 B1**
(45) Date of publication and mention of the grant of the patent: **09.07.2014**
(21) Application number: 07729500.4
(22) Date of filing: 24.05.2007
(51) Int. Cl.: C07H 19/06, C07H 19/16, A61P 29/00, A61P 27/02, A61P 25/00, A61P 11/00, A61K 31/7052

(54) **ECTONUCLEOTIDASE INHIBITORS**
EKTONUKLEOTIDASEINHIBITOREN
INHIBITEURS D'ECTONUCLÉOTIDASES

(30) Priority: 24.05.2006 EP 06114534
(43) Date of publication of application: 04.02.2009
(73) Proprietor: Rheinische Friedrich-Wilhelms-Universität Bonn, 53113 Bonn (DE)
(72) Inventor: MÜLLER, Christa E., 53113 Bonn (DE); BRUNSCHWEIGER, Andreas, 53225 Bonn-Beuel (DE); IQBAL, Jamshed, 53119 Bonn (DE)
(74) Representative: Von Kreisler Selting Werner - Partnerschaft von Patentanwälten und Rechtsanwälten mbB
(86) International application number: PCT/EP2007/055071
(87) International publication number: WO 2007/135195

(56) References cited:
- EP-A- 0 479 640
- EP-A1- 1 860 113
- WO-A-2006/121856
- GOPALAKRISHNAN, B. ET AL: "A Virtual Screening Approach for Thymidine Monophosphate Kinase Inhibitors as Antitubercular Agents Based on Docking and Pharmacophore Models" JOURNAL OF CHEMICAL INFORMATION AND MODELING, vol. 45, no. 4, 2005, pages 1101-1108, XP002443827 -& B. GOPALAKRISHNAN ET AL: "A Virtual Screening Approach for Thymidine Monophosphate Kinase Inhibitors as Anti-Tubercular Agents Based on Docking, Pharmacophore and 3D-QSAR Methods - Supporting Information" JOURNAL OF CHEMICAL INFORMATION AND MODELING, [Online] 2005, XP002443828 Retrieved from the Internet: URL:http://pubs.acs.org/subscribe/journals /jcisd8/suppinfo/ci050064z/ci050064zsi2005 0411_114335.pdf> [retrieved on 2007-07-24]
- YOUSSIF, SHAKER ET AL: "Synthesis of some new cytidine and inosine derivatives" BULLETIN OF THE KOREAN CHEMICAL SOCIETY , 26(12), 2021-2026 CODEN: BKCSDE; ISSN: 0253-2964, 2005, XP002443829
- DATABASE CA [Online] CHEMICAL ABSTRACTS SERVICE, COLUMBUS, OHIO, US; MOYER, JAMES D. ET AL: "Inhibition of uridine kinase and the salvage of uridine by modified pyrimidine nucleosides" XP002443831 retrieved from STN Database accession no. 1986:28510 & MOLECULAR PHARMACOLOGY , 28(5), 454-60 CODEN: MOPMA3; ISSN: 0026-895X, 1985,
- JIE L ET AL: "5'-O-Phosphonomethyl-2',3'-dideoxynucleos ides: Synthesis and anti-HIV activity" JOURNAL OF MEDICINAL CHEMISTRY 1990 UNITED STATES, vol. 33, no. 9, 1990, pages 2481-2487, XP002417215 ISSN: 0022-2623
- GENDRON F-P ET AL: "Novel inhibitors of nucleoside triphosphate diphosphohydrolases: Chemical synthesis and biochemical and pharmacological characterizations" JOURNAL OF MEDICINAL CHEMISTRY, AMERICAN CHEMICAL SOCIETY. WASHINGTON, US, vol. 43, no. 11, 1 June 2000 (2000-06-01), pages 2239-2247, XP002251346 ISSN: 0022-2623 cited in the application
- URI A ET AL: "A new class of compounds, peptide derivatives of adenosine 5'-carboxylic acid, includes inhibitors of ATP receptor-mediated responses." BIOORGANIC & MEDICINAL CHEMISTRY OCT 1994, vol. 2, no. 10, October 1994 (1994-10), pages 1099-1105, XP002417214 ISSN: 0968-0896

## Description

The present invention provides ectonucleotidase inhibitors including ecto-nucleotide triphosphate diphosphohydrolase (NTPDase) inhibitors and ecto-5'-nucleotidase (ecto-5'-NT) inhibitors, namely nucleotide mimetics as selective NTPDase or ecto-5'-NT inhibitors. It also provides methods for preparations of said compounds. Furthermore provided are pharmaceutical and diagnostic compositions comprising said compounds, and the use of said compounds in a medicament for treating diseases associated with ectonucleotidase activity and/or P1 or P2 receptors.

### Background of the Invention

Extracellular nucleotides such as ATP, ADP, UTP, and UDP can act as activators/agonists on a variety of nucleotide receptors (P2 receptors), namely purine P2 receptors and/or pyrimidine P2 receptors (Ralevic, V., and Burnstock, G., Pharmacol Rev 1998; 50: 413-92). The activation of P2 receptors is controlled by ecto-nucleotidases (NTPDases) capable of hydrolyzing nucleoside tri- and diphosphates (Zimmermann, H., Naunyn Schmiedebergs Arch Pharmacol 2000; 362: 299-309). Inhibition of ecto-nucleotidases can result in a potentiation of purinergic signaling, supporting the notion that endogenous ecto-nucleotidases reduce the effective concentration of the released nucleotide (Crack, B.E. et al., Br J Pharmacol 1994; 113: 1432-8; Crack, B.E. et al., Br J Pharmacol 1995; 114: 475-81; Bültmann, R. et al., Naunyn Schmiedebergs Arch Pharmacol 1995; 351: 555-60). Similarly, metabolically stable analogs of ATP are considerably more effective in causing a biological response than ATP itself (for references see Zimmermann, H., Ecto-nucleotidases. In Abbracchio, M.P. and Williams, M. (eds): Handbook of Experimental Pharmacology. Purinergic and Pyrimidergic Signalling, Heidelberg: Springer Verlag 2001; 209-50). Inhibitors of ecto-nucleotidases could thus represent valuable tools for amplifying the biological effects induced by extracellularly released nucleotides. In addition, inhibition of ecto-nucleotidases is mandatory for both, studies of nucleotide release and the analysis of the potency on P2 receptors of nucleotides or their hydrolyzable analogs. In addition, ectonucleotidase inhibitors (E-NTPDase as well as ecto-5'-NT inhibitors) inhibit the formation of adenosine, and thus the activation of adenosine receptors (P1 receptors).
P1 or adenosine receptors are subdivided into four distinct subtypes, A₁, A_{2A}, A_{2B}, and A₃ all of which are G protein-coupled receptors (Fredholm, B.B. et al., Pharmacol. Rev. 2001; 53: 527-552).
P2 receptors are divided in two categories: G protein-coupled receptors, termed P2Y (currently known subtypes: P2Y_{1,} P2Y_{2,} P2Y₄, P2Y₆, P2Y_{11,} P2Y_{12,} P2Y₁₃, P2Y₁₄) and ligand-gated cation channels, termed P2X (currently known subtypes: P2X₁₋₇). Several subtypes have been cloned within each family, and function in such systems as the central and peripheral nervous systems, the cardiovascular system, the endocrine system, lung, intestines, muscle, and the immune system (Xu, B. et al., J. Med. Chem. 2002; 45: 5694-5709; Fredholm, B.B. et al., Trends Pharm. Sci. 1997; 18: 79-82; Di Virgilio, F. et al., Blood 2001; 97: 587-600; Burnstock, G. and Williams, M., J. Pharmacol. Exp. Ther. 2000; 295: 862-869).

Inhibitors cf ecto-nucleotidases should have no effect on P1 or P2 receptors and should not be dephosphorylated by ecto-nucleotidase. Ideally they would also reveal selectivity for individual NTPDase isoforms or ecto-5'-NT. Many inhibitors of ecto-nucleotidases also act as antagonists of P2 receptors. These include suramin, pyridoxalphosphate-6-azophenyl-2',4'-disulfonic acid (PPADS) and reactive blue 2 (see Scheme 1 below; for references see Zimmermann, H., Ecto-nucleotidases. In Abbracchio, M.P. and Williams, M. (eds): Handbook of Experimental Pharmacology. Purinergic and Pyrimidergic Signalling, Heidelberg: Springer Verlag 2001; 209-50): NTPDase 2, for example, is predominantly expressed by hippocampal, cortical and cerebellar astrocytes. The enzyme probably modulates inflammatory reactions in the CNS and may therefore represent a useful therapeutic target in human diseases.

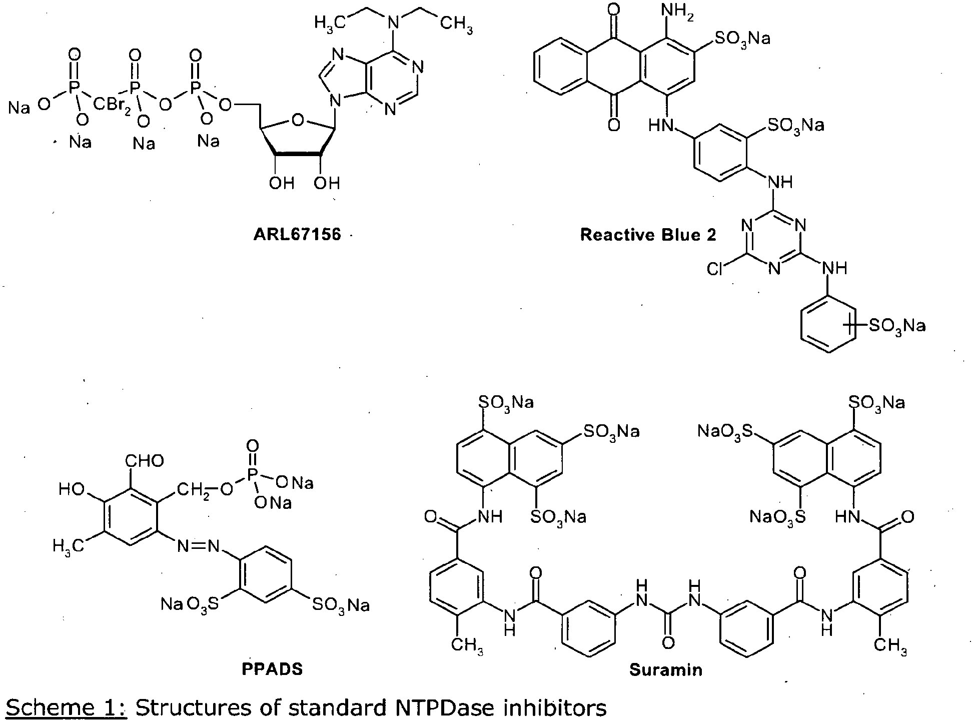

To date only the ATP analog ARL67156 (FPL67156, N⁶-Diethyl-β,γ-dibromomethylene-ATP; see Fig.1) (Crack, B.E. et al., Br J Pharmacol 1995; 114: 475-81; Kennedy, C. et al., Semin Neurosci 1996; 8: 195-99) and 8-thiobutyladenosine 5'-triphosphate (8-Bu-S-ATP) (Gendron, F.P. et al., J Med Chem 2000; 43: 2239-47) reveal enzyme inhibitory potential without significantly affecting nucleotide receptors. However, these compounds are not very potent, they are highly polar, and - since they contain phosphoric acid ester bonds - will probably not be highly stable but be hydrolyzed by physiological enzymes, such as ecto-nucleotide phosphatases (E-NPPs).

The ecto-nucleoside triphosphate diphosphohydrolases (EC 3.6.1.5) represent a major and ubiquitous family of ecto-nucleotidases. They catalyze the sequential hydrolysis of the γ- and β-phosphate residues of nucleoside tri- and diphosphates, producing the corresponding nucleoside monophosphate derivatives (Zimmermann, H., Naunyn Schmiedebergs Arch Pharmacol 2000; 362: 299-309). To date four different cell surface-located isoforms of the enzyme family have been cloned and functionally characterized (NTPDase1, 2 and 3, and very recently NTPDase8 (Bigonnesse, F. et al., Biochemistry 2004; 43: 5511-9; Zimmermann, H., Drug Dev Res 2001; 52: 44-56; Kukulski, F. et al., Purinergic Signalling 2005; 1: 193-204). The four enzymes differ in substrate specificity and in the pattern of product formation. Whereas NTPDase1 hydrolyzes ATP and ADP about equally well, NTPDase2 has a high preference for the hydrolysis of ATP over ADP. NTPDase3 and NTPDase8 are functional intermediates. NTPDase1 hydrolyzes ATP directly to AMP, ADP is the preferential product of ATP hydrolysis by NTPDase2, and NTPDase3 and NTPDase8 hydrolyze ADP formed from ATP efficiently to AMP. The different isoenzymes show distinct expression profiles.

Ecto-5'-nucleotidase (ecto-5'-NT, CD73, EC 3.1.3.5) is attached via a glycosylphosphatidylinositol (GPI) anchor to the plasma membrane, where it catalyzes the hydrolysis of nucleoside 5'-monophosphates such as AMP, GMP, or UMP to the respective nucleosides. ATP and ADP are competitive inhibitors of AMP hydrolysis (H. Zimmermann, Biochem. J. 1992, 285: 345-365). The main physiological function of ecto-5'-NT is the hydrolysis of extracellular AMP formed by the degradation of the P2 receptor agonists ATP and ADP by other ecto-nucleotidases. Thus, the enzyme generates adenosine, which can act on P1 (adenosine) receptors (N. Sträter, Purinergic Signalling 2006, 2, 343-350). Adenosine exerts multiple actions throughout the body; In human airways, adenosine is also mainly formed by the activity of ecto-5'-NT, in addition to a minor contribution by alkaline phosphatase (M. Picher et al., J. Biol. Chem. 2003, 278, 13468-13479).. Recently, ecto-5'-NT knock-out mice have been generated, which showed increased leukocyte adhesion in the vascular endothelium after ischemia-reperfusion (Koszalka P. et al., Circ. Res. 2004, 95, 814-821). These findings point to an important role of ecto-5'-NT in tissue inflammation and immune responses. Ecto-5'-NT as well as NTPDase1 have been reported to be highly expressed in melanoma cells, and the ecto-5'-NT level has been associated with their ability to metastasize (Sadej R. et al., Melanoma Res. 2006, 16, 213-222; Dzhandzhugazyan, K.N. et al., FEBS Lett. 1998, 430, 227-230). Ectonucleotidases and adenosine are involved in immune responses, e.g. involving T-cells and B-cells (Resta, R. et al., Immunol. Rev. 1998, 161: 95-109), and in tumor promotion (Spychala J., Pharmacol. Ther. 2000, 87, 161-173).

Potential therapeutic applications of NTPDase inhibitors include all disease therapies which aim at increasing the nucleotide concentration or reducing the adenosine concentration in a patient, while therapeutic applications of ecto-5'-NT inhibitors include disease therapies which aim at reducing adenosine concentrations (Ralevic, V., and Burnstock, G., Pharmacol Rev 1998; 50: 413-92; Brunschweiger, A. and Müller, C.E., Curr. Med. Chem. 2006, 13, 289-312; Vekaria, R. M. et al., Am. J Physiol Renal Physiol 2006, 290, F550-F560; Gendron, F.P. et al., Curr Drug Targets 2002, 3, 229-245; Sträter, N. Purinergic Signalling, 2006, 2, 343-350). Potential applications include dry eye disease (local application), respiratory diseases, cystic fibrosis, inflammatory diseases, diseases of the immune system, gastrointestinal diseases, kidney disorders, cancer, and brain diseases. Furthermore, selective NTPDase inhibitors and ecto-5'-NT inhibitors may be useful for diagnostic purposes and as pharmacological tools.

For example, NTPDase2 is predominantly expressed by hippocampal, cortical and cerebellar astrocytes. The enzyme probably modulates inflammatory reactions in the CNS and therefore represents a potential therapeutical target (Wink, M.R. et al., Neuroscience 2006, 138, 421-432).

However, so far no highly potent and at the same time highly selective inhibitors for certain subtypes of NTPDases have been described. Such compounds could increase extracellular nucleotide concentrations in an event- and site-specific manner and thus act as indirect, P2 receptor agonists. Selective NTPDase inhibitors should not exhibit affinity for P2 receptors. NTPDase inhibitors showing the desired properties may be used as novel therapeutics (drugs) for various diseases.

Up to now it has mainly been attempted to develop direct P2 receptor agonists. Major drawbacks of such compounds are (i) that they do not act in a site- and event-specific manner, but at all receptors, and not only where the nucleotide concentration is high; (ii) the P2 agonists that are known so far are highly polar containing negative charges, since they are derived from nucleotides. Therefore they are only parenterally applicable.

For ecto-5'-NT only very few inhibitors have been described to date (Zimmermann H., Biochem. J. 1992, 285, 345-365). The standard inhibitor is an analog of ADP, in which the β-phosphate ester bond is replaced by a methylene group (β-methylene-ADP, AOPCP). The compound is a nucleotide analog bearing negative charges at physiologic pH value.

On the other hand, certain 5' derivatives of adenosine acid were described in the following:
Uri A. et al. Bioorganic & Medical Chemistry, Vol. 2, No. 10, pp. 1099-1105 (1994) and Kawana M. et al., J. Org. Chem., Vol. 37, No. 2, pp. 288-291 (1972) disclose conjugates of amino acids and adenosine 5' carboxylic acids.
US 3,914,415 discloses adenosine-5' carboxylic acid amindes.
Further, Jie L. et al., J. Med. Chem. 33:248, 2481-2487 (1990) discloses 5' O-Phosphonomethyl-2'3' dideoxy nucleosided and Walker, T.E. et al., Carbohydrate Res.. 27, 225-234 (1993) discloses 5'-C-alkyl analogues of adenosine.
Finally, WO 2006/121856 (published November 16, 2006) discloses 4-aminoacyl pyrimidine nucleoside analogues carrying a 5' carbon chain.
Thus, the problem underlying present invention is the provision of isoenzyme-selective ectonucleotidase inhibitors, namely NTPDase and ecto-5'-NT inhibitors, which are not highly polar, do not block P2 receptors and which preferably act in a site and event specific manner.

### Summary of the invention

The present invention provides new class of ectonucleotidase inhibitors, namely NTPDase and ecto-5'-NT inhibitors, which are not nucleotides, but nucleotide mimetics. Preferably, said compounds are neutral (not anionic/ negatively charged). The compounds are selective versus P2 receptors and exhibit high potency to inhibit ectonucleotidases and some are selective for certain NTPDase subtypes or ecto-5'-NT. The compounds are derivatives of nucleosides or nucleoside derivatives; they can be described as nucleotide mimetics, in which the phosphate chain of the corresponding nucleotides is replaced by various substituents of different lengths, e.g. bearing a terminal phosphonic acid diester group. The nucleobase is an oxopurin or oxopyrimidin that can be derivatized or otherwise modified. The ribose moiety can also be modified. The compounds show peroral bioavailability and, in contrast to nucleotides, are metabolically considerably more stable. The compounds are competitive inhibitors of NTPDases or ecto-5'-NT, respectively and are suitable for the treatment of a number of different diseases in which the activation of P2 receptors and/or the inhibition of activation of adenosine receptors is advantageous.

Thus, the present invention provides
(1) a compound represented by the formula (I) 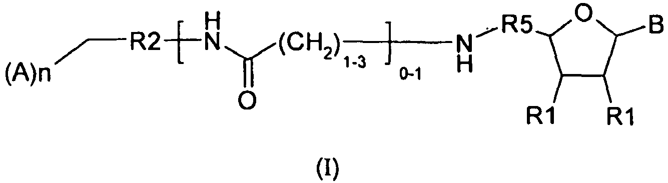 wherein
   B represents an oxopurinyl or oxopyrimidinyl residue which is connected with the furanoside ring via one of its nitrogen atoms;
   R1 represent independently from each other residues selected from hydroxyl, hydrogen, C₁-C₃-alkoxyl, C₁-C₃-alkyl, C₁-C₃-alkenyl, C₁-C₃-alkinyl, C₁-C₃-acyl, halogen, or commonly form a double bond with one of the vicinal C atoms or an acetyl or ketal ring with each other;
   R2 is -(CH₂)₀₋₂- or phenylene;
   A represents a -PO(OR3)₂ or -SO2(OR3) residue wherein R3 is C₁-C₃-alkyl, aryl, arylalkyl or heteroaryl or A represents a -(CH₂)ₘ-COOR4 residue, wherein m is an integer from 0 to 2 and R4 is selected from hydrogen and C₁-C₃-alkyl;
   R5 is a carbonyl or a methylene group; and n is 1 or 2, provided that when A represents a -(CH₂)ₘ-COOR4 residue, n is 2; or a salt thereof.
(2) a pharmaceutical or diagnostic composition or a medicament comprising a compound as defined in (1) above;
(3) the use of the compound as defined in (1) above for the preparation of a medicament for treating diseases connected with a reduced abundance of nucleotides in a patient or for therapies aiming at increasing the nucleotide concentration in a patient;
(4) the use of the compound as defined in (1) above as selective NTPDase inhibitor;
(5) an *in vitro* method for ATP quantification using the compound as defined in (1) above; and
(6) a method for preparing the compound as defined in (1) above.

### Detailed Description of Invention

The compounds of present invention are structurally derived from nucleosides. In their broadest sense, they can be seen as nucleotide-mimetics wherein the phosphate chain is replaced with moieties which are less prone to hydrolysis, namely by a carbohydrate chain forming an amide or amine with the ribose on one end and bearing an ester or acid group on the other end as shown in the compound of formula (I) set forth above.

In more detail, in the formula (I) representing the compound of embodiment (1) the variables are defined as follows:
B represents an oxopurinyl or an oxopyrimidinyl residue. Said residue is preferably a native oxopurinyl or oxopyrimidyl including uracilyl, thyminyl, cytosinyl and methylcytosinyl, guaninyl, hypoxanthinyl or xanthinyl (but is not an adenosyl residue), most preferably is an uracilyl residue.
B is connected with the ribose moiety via one of the ring nitrogen atoms, preferably via the N-1 of the pyrimidinyl residues or the N-9 of the purinyl residues. More preferably, B is 1-uracilyl.
R1 represent independently from each other residues selected from the group consisting of hydroxyl, hydrogen, C₁-C₃-alkoxyl, C₁-C₃-alkyl, C₁-C₃-alkenyl, C₁-C₃-alkinyl, C₁-C₃-acyl, halogen, or commonly form a double bond with one of the vicinal C atoms or an acetyl or ketal ring with each other. Preferably, at least one R1 is OH and the other R1 is H or OH. More preferred, both R1 are OH.
R2 is -(CH₂)₀₋₂- or phenylene. If R2 is phenylene, it may be connected in o-, m- or p-position with the other elements of the compound according to present invention. However, the p-connection is preferred.
R5 is a carbonyl or methylidene (-CH₂-) group. It is preferably a carbonyl group, thus forming an amide bound with the adjacent amine function.

Moreover, it is preferred that the spacer molecule, i.e. the atoms between the 5'C atom of the nucleotide and the acidic moiety A is at least three carbon or hetereatoms (O, N or S).

The ring atoms of the ribose unit are chiral. The spatial orientation of their substituents is arbitrary. However, an orientation like in the native ribose furanoside of nucleotides is preferred. Said orientation is the one represented in the following formula of a preferred compound of present invention: 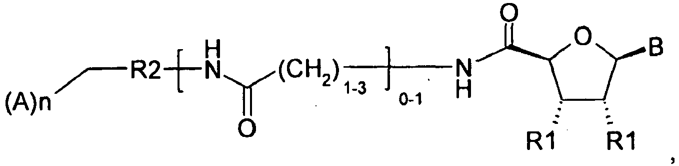 wherein all variables are defined as above.
A represents a -PO(OR3)₂ or -SO2(OR3) residue, wherein m is an integer from 0 to 2, R3 is C₁-C₃-alkyl, aryl, arylalkyl (e.g. benzyl) or heteroaryl or represents a -(CH₂)ₘ-COOR4 residue, wherein R4 is selected from hydrogen and C₁-C₃-alkyl,
n is 1 or 2, provided that when A represents a -(CH₂)ₘ-COOR4 residue, n is 2;
or a salt thereof.

In one preferred aspect of the invention, A represents a -PO(OR3)₂ residue. If n is 1, this means that there is one terminal -PO(OR3)₂ group in the compound of present invention. If n is 2, there are two of them. However, it is preferred that n is 1. Furthermore in said preferred aspect, R3 is preferably an ethyl or methyl moiety, most preferably an ethyl moiety.

Thus, an especially preferred compound of present invention is represented by the following formula: 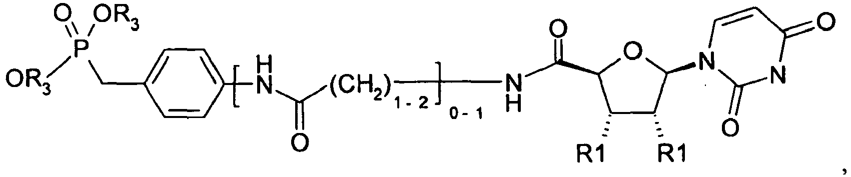 wherein preferably
(i) at least one R1 is OH and the other R1 is H or OH, more preferably both R1 are OH; and/or
(ii) R3 is ethyl.

As far as residue A is concerned, in a further preferred aspect of present invention said residue A represents a -(CH₂)ₘ-COOR4 residue. In this aspect, moreover, R4 is H and n is 2. Even more preferred, m is 0 in one of the two -(CH₂)ₘ-COOR4 groups.

The following compounds of embodiment (1) are especially preferred (hereinafter referred to as "Compounds (1) to (26) of the invention"):

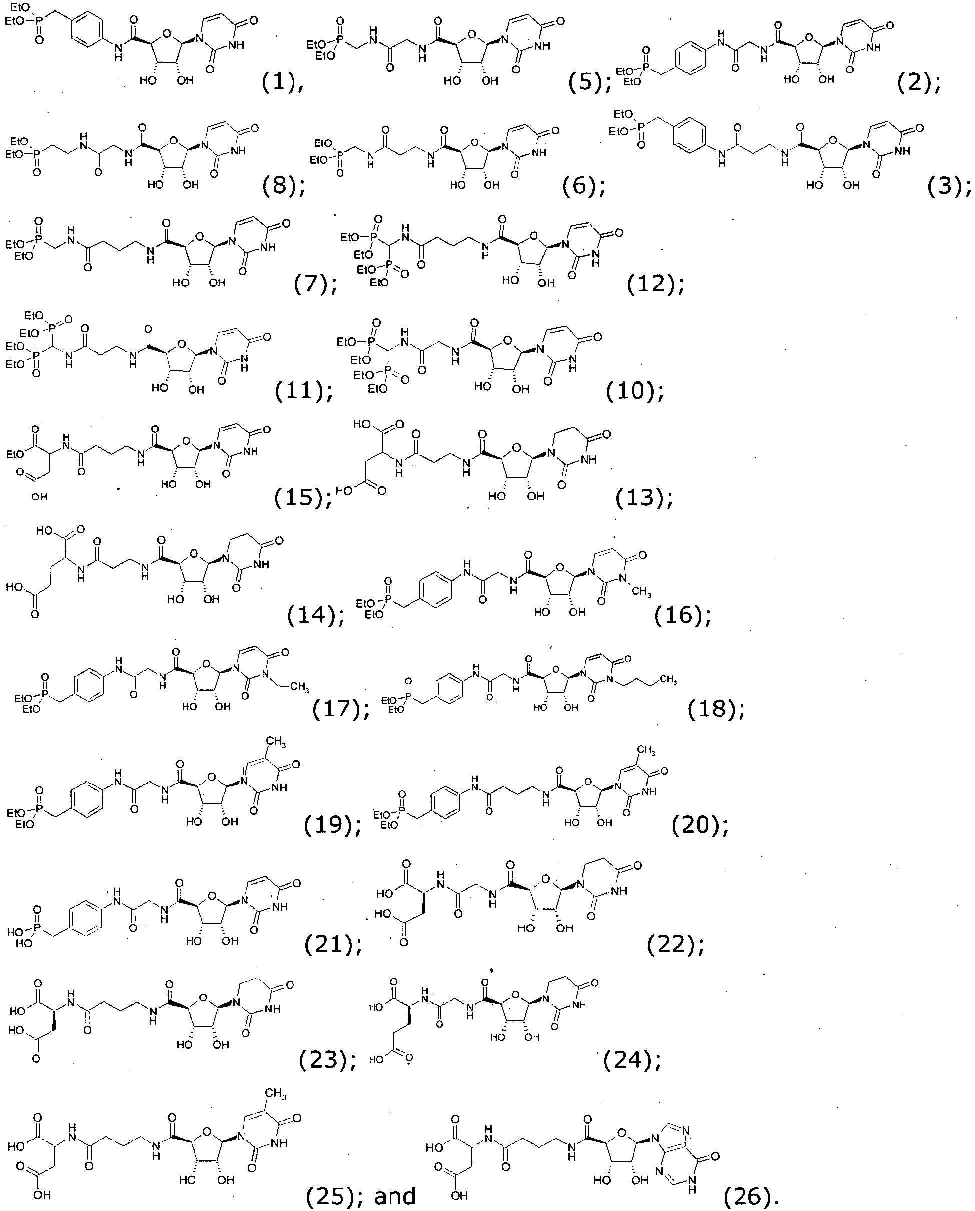

Among these compounds, the compound which is represented by the formula 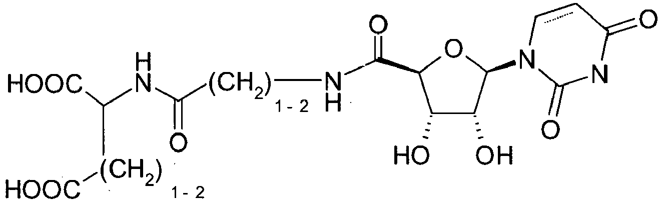 namely compounds (13), (14), (22) and (24), the compound which is represented by the formula 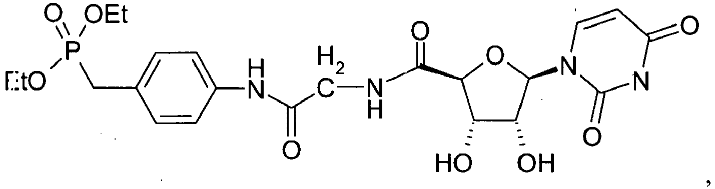 namely compound (2), are the most preferred ones. The latter one is an excellent inhibitor of NTPDase (compare Tab. 1) and is therefore even more preferred.

The compounds of present invention are probably competitive inhibitors of NTPDases. Thus, they are of interest for any therapy wherein an activation of P2 receptors is advantageous.

The pharmaceutical composition of embodiment (2) is preferably the medicament of embodiment (3). Furthermore, said medicament of embodiment (3) is preferably for therapy of dry eye disease, respiratory diseases, cystic fibrosis, inflammatory diseases, diseases of the immune system, gastrointestinal diseases, kidney disorders, cancer, and brain diseases. Especially preferred is a medicament for therapy of cancer.

The pharmaceutical composition and the medicament of present invention are applicable in any way allowing the incorporation of the compounds of present invention. As the compounds of present invention are more stable to hydrolysis than compounds containing a phosphate chain, their oral application is preferred.

A further preferred aspect of present invention is the use of the compounds of embodiment (1) in the method of embodiment (5). Especially preferred is the use in a luciferase assay. The known NTPDase inhibitor ARL 67156 is metabolically unstable towards ecto-nucleotide pyrophosphatases (E-NPP). It can be applied as a pharmacological tool but is not suitable in assays where the luciferase assay is used for the quantification of ATP concentrations since it interferes with that assay. It was shown that the compounds of embodiment (1) do not interfere with the luciferase assay for ATP determination. They have therefore major advantages as pharmacological tools in comparison to ARL 67156 and other known NTPDase inhibitors.

The method (6) preferably comprises the following steps: reacting a compound of formula (II) 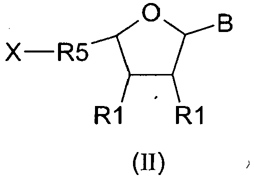 wherein X is a leaving group and all other variables are as defined above, with a compound of formula (III) 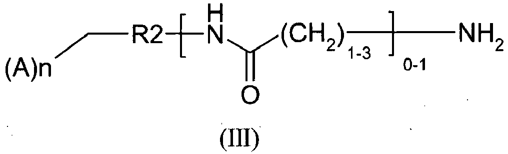 wherein all variables are as defined above. Of course, reactive groups which are not part of said coupling reaction (e.g. the free hydroxy groups of the ribose moiety) are adequately protected beforehand and deprotected after the reaction. Such protection/deprotection reactions are known in the art and exemplified in examples 1 to 20.

The leaving group X is selected from halogen, tosylate, mesylate, and activated esters.

The present invention is described in more detail by reference to the following examples. It should be understood that these examples are for illustrative purpose only and are not to be construed as limiting the invention.

### Examples

All commercially available chemicals and solvents were obtained from various companies (Fluka, Merck, Acros, Sigma-Aldrich). Preparative column chromatography was performed on silica gel 60 (Fluka) 230-400 mesh. Preparative RP-HPLC was performed on a Eurosphere 100 C₁₈ column (250 x 20 mm) with a mixture of MeOH and H₂O at a flow rate of 20 ml/min. ¹H-NMR-, ¹³C-NMR- and ³¹P-NMR-spectra were recorded on a Bruker Avance 500 NMR-spectrometer. Shifts (δ) are given in ppm. The ESI mass spectra were recorded on an API 2000 (Applied Biosystems, Darmstadt, Germany) mass spectrometer at the Pharmaceutical Institute Poppelsdorf, University of Bonn, Germany (ESI, sprayed from a 10⁻⁵ M solution in 2 mM NH₄OAc/MeOH 0.75:0.25, flow rate 10 µl/min).

### Example 1: Synthesis of the Ectonucleotidase Inhibitors and Comparative Compounds (CC)

### A. 4-[(2S,3R,4S,5R)-(2,4-Dioxo-3,4-dihydropyrimidin-1(2H)-yl)-3,4-dihydroxy-tetrahydrofurane-2-carboxamido]benzylphosphonic acid diethylester (1)

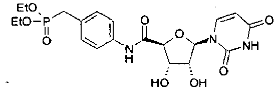

Under an atmosphere of argon, 2',3'-anisylideneuridine-4'-carboxylic acid (1 mmol, 376 mg), PyBOP^{®} (1.1 mmol, 572 mg) and 1-hydroxybenzotriazole (1.1 mmol, 149 mg) were dissolved in 2 ml of dry DMF at r.t. Diisopropylethylamine (1.1 mmol, 143 mg) and, after one minute, aminobenzylphosphonic acid diethyl ester (2 mmol, 1215 mg), dissolved in 2 ml of dry DMF, were added sequentially via a syringe to the solution. Vigorous stirring was continued for 24 hours at ambient temperature. The volatiles were removed in vacuum at 40 °C and the residue purified by silica gel column chromatography using dichloromethane:methanol (40:1) as an eluent. The product was isolated by rotary evaporation at 40 °C and recrystallized from diethyl ether.

Deprotection of the ribose moiety was performed by stirring 2',3'-anisylideneuridine-5'-amide (100 mg) in a mixture of dichloromethane (3 ml), trifluoroacetic acid (0.15 ml) and water (0.1 ml) at ambient temperature. After two hours, the crude product was precipitated by the addition of diethyl ether (50 ml), filtered off, dissolved in 7 ml of water/methanol (75:25) and purified by RP-HPLC using a gradient of water/methanol of 75:25 to water/methanol 0:100. 230 mg of the title compound (1) was obtained by lyophilization as white amorphous powder (yield over two steps: 48%).
¹H-NMR (500 MHz, MeOD), δ (ppm) 8.17 (d, 1H, ³J = 7.90 Hz, H-6), 7.67 (d, 2H, ³J = 8.85 Hz, 2 x CHₒᵣₜₕₒ, benzyl phosphonate), 7.32 (dd, 2H, ³J = 8.80 Hz and ⁴J = 2.80 Hz, 2 x CHₘₑₜₐ, benzyl phosphonate), 5.85 (d, 1H, ³J = 6.30 Hz, H-1'), 5.80 (d, 1H, ³J = 8.20 Hz, H-5), 4.61 (dd, 1H, ³J = 5.05 Hz and ³J = 5.95 Hz, H-2'), 4.56 (d, 1H, ³J = 3.20 Hz, H-4'), 4.35 (dd, 1H, ³J = 3.20 Hz and ³J = 5.05 Hz, H-3'), 4.10 - 4.04 (2 x q, 4H, 2 x O-CH₂), 3.26 (d, 2H, ²J_{H,P} = 21.45 Hz, CH₂P, benzyl phosphonate), 1.30 (t, 6H, 2 x CH₃).
¹³C-NMR (125 MHz, MeOD), δ (ppm) 170.8 (C=O), 166.5 (C-4), 153.1 (C-2), 145.2 (C-2), 138.5 (Cₚₐᵣₐ, benzyl phosphonate), 131.7 (2 x CHₒᵣₜₕₒ, benzyl phosphonate), 129.2 (d, ²J_{C,P} = 38.7 Hz, Cᵢₚₛₒ, benzyl phosphonate), 121.7 (2 X CHₘₑₜₐ, benzyl phosphonate), 103.5 (C-5), 94.1 (C-1'), 86.1 (C-4'), 75.1 (C-2'), 73.8 (C-3'), 64.1 (2 x O-CH₂), 33.5 (d, ¹J_{C,P} = 551.2 Hz, CH₂-P, benzyl phosphonate), 17.0 (2 x CH₃).
³¹_{P}-_{NMR} (202 MHz, MeOD) δ (ppm) 26.7.
MS (ESI), m/z +1: 484.1; m/z -1: 482.3.

### B. 4-[2-((2S,3R,4S,5R)-5-(2,4-Dioxo-3,4-dihydropyrimidin-1(2H)-yl)-3,4-dihydro-xy-tetrahydrofurane-2-carboxamido)ethaneamido]benzylphosphonic acid diethyl-ester 2

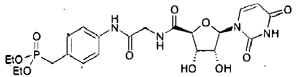

In a dry vessel, N-tert-butyloxycarbonylglycine (10 mmol, 1750 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) was added. The resulting mixture was allowed to warm to ambient temperature. After three hours, the volatiles were removed by rotary evaporation at 40 °C, the residue was dissolved in 10 ml of water and adjusted to pH 1 (10 % aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. p-(Aminomethylcarboxamido)benzylphos-phonic acid diethyl ester hydrochloride was precipitated by addition of 50 ml of diethyl ether, filtered off and thoroughly washed with diethyl ether (yield over two steps: 3100 mg, 92 %, white crystals).

Under an atmosphere of argon, 2',3'-anisylideneuridine-4'-carboxylic acid (1 mmol, 376 mg), HCTU^{®} (1.1 mmol, 455 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, p-(aminomethylcarboxamido)benzylphosphonic acid diethyl ester hydrochloride (2 mmol, 672 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneuridine-5'-amide (100 rng) in a mixture of dichloromethane (3 ml), trifluoroacetic acid (0.15 ml) and water (0.1 ml) at ambient temperature. After two hours, the crude product was precipitated by the addition of diethyl ether (50 ml), filtered off, dissolved in 7 ml of water/methanol (75:25) and purified by RP-HPLC using a gradient of water/methanol from 75:25 to water/methanol 0:100. 310 mg of the title compound (2) was obtained by lyophilization as white amorphous powder (yield over two steps: 57%).
¹H-NMR (500 MHz, MeOD), δ 8.18 (d, 1H, ³J = 7.90 Hz, H-6), 7.58 (d, 2H, ³J = 8.85 Hz, 2 x CH*ₒᵣₜₕₒ*, benzyl phosphonate), 7.31 (dd, 2H, ³J = 8.80 Hz and ⁴J = 2.80 Hz, 2 x *CHₘₑᵣₐ,* benzyl phosphonate), 6.02 (d, 1H, ³J = 6.30 Hz, H-1'), 5.78 (d, 1H, ³J = 8.20 Hz, H-5), 4.51 (d, 1H, ³J = 3.20 Hz, H-4'), 4.47 (dd, 1H, ³J = 5.05 Hz und ³J = 5.95 Hz, H-2'), 4.41 (dd, 1H, ³J = 3.20 Hz and ³J = 5.05 Hz, H-3'), 4.19 - 4.01 (AB-system with A d and B d, partially overlapping with 2 x O-CH₂, 2H, ²J = 16.35 Hz, N-CH₂, ethaneamide), 4.09 - 4.01 (2 x q, 4H, 2 x O-CH₂), 3.25 (d, 2H, ²J_{H,P} = 21.45 Hz, CH₂-P, benzyl phosphonate), 1.29 (t, 6H, 2 x CH₃).
¹³C-NMR (125 MHz, MeOD) δ 173.2 (C=O), 169.5 (C=O), 166.4 (C-4), 153.1 (C-2), 144.3 (C-6), 138.9 (C*ₚₐᵣₐ*, benzyl phosphonate), 131.7 (2 x CH*ₒᵣₜₕₒ*, benzyl phosphonate), 128.7 (d, ²J_{C,P} = 9.4 Hz, C*ᵢₚₛₒ*, benzyl phosphonate), 121.5 (2 x CH*ₘₑₜₐ*, benzyl phosphonate), 103.5 (C-5), 92.2 (C-1'), 85.3 (C-4'), 74.9 (C-2'), 74.1 (C-3'), 64.1 und 64.0 (2 x O-CH₂), 43.9 (N-CH₂, ethaneamide), 33.4 (d, ¹J_{C,P} = 137.6 Hz, CH₂-P, benzyl phosphonate), 17.0 und 16.9 (2 x CH₃).
³¹P-NMR (MeOD) δ 26.7.
MS (ESI), m/z +1: 541.0; m/z -1: 539.3.

### C. 4-[3-((2S,3R,4S,5R)-5-(2,4-Dioxo-3,4-dihydropyrimidin-1(2H)-yl)-3,4-di-hydroxy-tetrahydrofurane-2-carboxamido)propaneamido]benzylphosphonic acid diethylester (3)

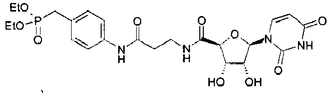

In a dry vessel, N-tert-butyloxycarbonyl-β-alanine (10 mmcl, 1890 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochlorice) a solution of aminobenzylphosphonic acid diethyl ester (11 mmol, 2673 mg) in dry THF (10 ml) was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. *p*-(2-Aminoethylcarboxamido)benzylphos-phonic acid diethyl ester hydrochloride was precipitated by addition of 50 ml of diethyl ether, filtered off and thoroughly washed with diethyl ether (yield over two steps: 3000 mg, 86%, white crystals).

Under an atmosphere of argon, 2',3'-anisylideneuridine-4'-carboxylic acid (1 mmol, 376 mg), HBTU^{®} (1.1 mmol, 428 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, p-(2-aminoethylcarboxamido)benzylphosphonic acid diethyl ester hydrochloride (2 mmol, 700 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane:methanol (40:1). The product was isolated by rotary evaporation at 40 °C and recrystallized from diethyl ether. Deprotection of the ribose moiety was performed by stirring 2',3'-anisylideneuridine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 350 mg of the title compound (3) was isolated by lyophilization as white amorphous powder (yield over two steps: 63%).
¹H-NMR (500 MHz, MeOD), δ 8.08 (d, 1H, ³J = 8.20 Hz, H-6), 7.55 (d, 2H, ³J = 7.90 Hz, 2 x CH*ₒᵣₜₕₒ*, benzyl phosphonate), 7.29 (dd, 2H, ³J = 8.50 Hz and ⁴J = 2.85 Hz, 2 x CH*ₘₑₜₐ*, benzyl phosphonate), 5.92 (d, 1H, ³J = 6.30 Hz, H-1'), 5.72 (d, 1H, ³J = 7.90 Hz, H-5), 4.41 (dd, 1H, ³J = 5.05 Hz and ³J = 6.30 Hz, H-2'), 4.40 (d, 1H, ³J = 2.85, H-4'), 4.28 (dd, 1H, ³J = 5.05 Hz and ²J = 2.85 Hz, H-3'), 4.10 - 4.03 (2 x q, 4H, 2 x O-CH₂), 3.69 - 3.56 (m, 2H, ³J = 6.30 Hz, N-CH₂, propaneamide), 3.23 (d, 2H, ²J_{H,P} = 21.45 Hz, CH₂-P, benzyl phosphonate), 2.65 (m, 2H, ³J = 6.30 Hz, O=C-CH₂, propaneamide), 1.29 (t, 6H, 2 x CH₃).
¹³C-NMR (125 MHz, MeOD) δ 172.6 (C=O), 172.2 (C=O), 166.3 (C-4), 152.9 (C-2), 144.3 (C-6), 139.1 (C*ₚₐᵣₐ*, benzyl phosphonate), 131.7 (2 x CH*ₒᵣₜₕₒ*, benzyl phosphonate), 128.6 (d, ²J_{C,P} = 9.4 Hz, C*ᵢₚₛₒ*, benzyl phosphonate), 121.5 (2 x CH*ₘₑₜₐ*, benzyl phosphonate), 103.4 (C-5), 92.4 (C-1'), 85.4 (C-4'), 74.9 (C-2'), 74.1 (C-3'), 64.1 and 64.0 (2 x O-CH₂), 36.6 (N-CH₂, propaneamide), 37.1 (O=C-CH₂, propaneamide), 33.4 (d, ¹J_{C,P} = 137.6 Hz, CH₂-P, benzyl phosphonate), 16.9 and 16.8 (2 x CH₃).
³¹P-NMR (202 MHz, MEOD) δ 26.8.
MS (ESI), m/z +1: 555.3; m/z -1: 553.3.

### D. 4-[4-((2S,3R,4S,5R)-5-(2,4-Dioxo-3,4-dihydropyrimidin-1(2H)-yl)-3,4-dihydroxy-tetrahydrofurane-2-carboxamido)butaneamido]benzylphosphonic acid diethyl-ester (4)

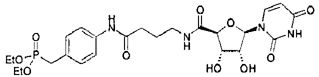

In a dry vessel, N-tert-butyloxycarbonyl-y-aminobutyric acid (10 mmol, 2030 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. p-(3-Aminopropylcarboxamido)benzyl-phosphonic acid diethyl ester hydrochloride was precipitated by addition of 50 ml of diethyl ether, filtered off and thoroughly washed with diethyl ether (yield over two steps: 3300 mg, 91%, white crystals).

Under an atmosphere of argon, 2',3'-anisylideneuridine-4'-carboxylic acid (1 mmol, 376 mg), HBTU^{®} (1.1 mmol, 482 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, p-(aminopropylcarboxamido)benzylphosphonic acid diethylester hydrochloride (2 mmol, 728 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneuridine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0: 100. 340 mg of the title compound (4) was obtained by lyophilization as white amorphous powder (yield over two steps: 60%).
¹H-NMR (500 MHz, MeOD), δ 8.11 (d, 1H, ³J = 8.20 Hz, H-6), 7.55 (d, 2H, ³J = 7.90 Hz, 2 x CH*ₒᵣₜₕₒ*. benzyl phosphonate), 7.28 (dd, 2H, ³J = 8.50 Hz and ⁴J = 2.85 Hz, 2 x CH*ₘₑₜₐ*, benzyl phosphonate), 5.86 (d, 1H, ³J = 6.30 Hz, H-1'), 5.75 (d, 1H, ³J = 7.90 Hz, H-5), 4.45 (dd, 1H, ³J = 5.05 Hz and ³J = 6.30 Hz, H-2'), 4.39 (d, 1H, ³J = 2.85, H-4'), 4.27 (dd, 1H, ³J = 5.05 Hz and ²J = 2.85 Hz, H-3'), 4.09-4.03 (2 x q, 4H, 2 x O-CH₂), 3.38 (t, partly below solvent peak, 2H, ³J = 7.25 Hz, N-CH₂, butaneamide), 3.23 (d, 2H, ²J_{H,P} = 21.45 Hz, CH₂-P, benzyl phosphonate), 2.45 (t, 2H, ³J = 7.25 Hz, O=C-CH₂, butaneamide), 1.95 (tt, 2H, ³J = 7.25 Hz, CH₂, butaneamide), 1.29 (t, 6H, 2 x CH₃).
¹³C-NMR (125 MHz, MeOD) δ 174.0 (C=O), 172.7 (C=O), 166.3 (C-4), 152.9 (C-2), 144.6 (C-6), 139.2 (C*ₚₐᵣₐ*, benzyl phosphonate), 131.6 (2 x CH*ₒᵣₜₕₒ*, benzyl phosphonate), 128.5 (d, ²J_{C,P} = 9.4 Hz, C*ᵢₚₛₒ*, benzyl phosphonate), 121.4 (2 x CH*ₘₑₜₐ*, benzyl phosphonate), 103.3 (C-5), 93.1 (C-1'), 85.4 (C-4'), 74.8 (C-2'), 74.1 (C-3'), 64.0 (2 x O-CH₂), 40.1 (N-CH₂, butaneamide), 35.5 (O=C-CH₂*,* butaneamide), 33.4 (d, ¹J_{C,P} = 137.6 Hz, CH₂-P, benzyl phosphonate), 26.6 (CH₂, butaneamide), 16.9 and 16.8 (2 x CH₃).
³¹P-NMR (202 MHz, MeOD) δ 26.8.
MS (ESI), m/z +1: 569.2; m/z -1: 567.3.

### E. 2-[(2S,3R,4S,5R)-5-(2,4-Dioxo-3,4-dihydropyrimidin-1(2H)-yl)-3,4-dihydroxy-tetrahydrofurane-2-carboxamidolethaneamidomethylphosphonic acid diethylester (5)

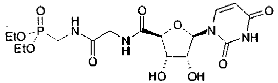

In a dry vessel, N-tert-butyloxycarbonylglycine (10 mmol, 1750 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added, under vigorous stirring. Immediately after the formation of a white precipitate (N-methylmorpholine hydrochloride) a solution of aminomethylphosphonic acid diethylester oxalate (11 mmol, 2827 mg) in THF (10 ml) and 1N aq. NaOH (11 ml), pre-cooled on ice, was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. Aminomethylcarboxamidomethylphosphonic acid diethyl ester hydrochloride was precipitated by addition of 50 ml of diethyl ether, filtered off and thoroughly washed with diethyl ether (yield over two steps: 2200 mg, 85%, white crystals).

Under an atmosphere of argon, 2',3'-anisylideneuridine-4'-carboxylic acid (1 mmol, 376 mg), HCTU^{®} (1.1 mmol, 455 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, aminomethylcarboxamidomethylphosphonic acid diethyl ester hydrochloride (2 mmol, 522 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneuridine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0: 100. 190 mg of the title compound (5) was isolated by lyophilization as white amorphous powder (yield over two steps: 41%).
¹H-NMR (500 MHz, MeOD), δ 8.10 (d, 1H, ³J = 7.85 Hz, H-6), 5.91 (d, 1H, ³J = 5.95 Hz, H-1'), 5.77 (d, 1H, ³J = 7.85 Hz, H-5), 4.50 (dd, 1H, ³J = 5.35 Hz and ³J = 5.70 Hz, H-2'), 4.48 (d, 1H, ³J = 2.85 Hz, H-4'), 4.44 (dd, 1H, ³J = 5.0 Hz and ³J = 3.15 Hz, H-3'), 4.22 - 4.15 (2 x q, 4H, 2 x O-CH₂), 4.10-3.83 (AB-system with A d and B d, 2H, ²J = 17.00 Hz, N-CH₂, ethaneamide), 3.84-3.72 (AB-system with A dd and B dd, 2H, ²J_{H,P} = 11.65 Hz and ²J = 15.75 Hz, N-CH₂, methyl phosphonate), 1.35 (2 x t, 6H, 2 x CH₃).
¹³C-NMR (125 MHz, MeOD) δ 173.2 (C=O), 171.6 (C=O), 166.4 (C-4), 153.1 (C-2), 144.9 (C-6), 103.5 (C-5), 93.9 (C-1'), 85.7 (C-4'), 74.9 (C-2'), 74.2 (C-3'), 64.5 (2 x O-CH₂), 43.4 (N-CH₂, ethaneamide), 35.7 (d, ¹J_{C,P} = 157.6 Hz, CH₂-P, methyl phosphonate), 17.0 (2 x CH₃).
³¹P-NMR (202 MHz, MeOD) δ 22.1.
MS (ESI), m/z +1: 465.1; m/z -1: 463.1.

### F. 3-1(2S,3R,4S,5R)-5-(2,4-Dioxo-3,4-dihydropyrimidin-1(2H)-yl)-3,4-dihydroxy-tetrahydrofurane-2-carboxamido]propaneamidomethylphosphonic acid diethylester (6)

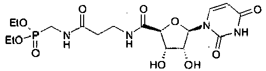

In a dry vessel, N-tert-butyloxycarbonyl-β-alanine (10 mmol, 1750 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of aminomethylphosphonic acid diethyl ester oxalate (11 mmol, 2827 mg) in THF (10 ml) and 1N aq. NaOH (11 ml), pre-cooled on ice, was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. 2-Aminoethylcarboxamidomethylphosphonic acid diethyl ester hydrochloride was precipitated by addition of 50 ml of diethyl ether, filtered off and thoroughly washed with diethyl ether (yield over two steps: 2000 mg, 73%, clay).

Under an atmosphere of argon, 2',3'-anisylideneuridine-4'-carboxylic acid (1 mmol, 376 mg), PyBOP^{®} (1.1 mmol, 455 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, 2-aminoethylcarboxamidomethylphosphonic acid diethyl ester hydrochloride (2 mmol, 578 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneuridine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 170 mg of the title compound (6) was isolated by lyophilization as white amorphous powder (yield over two steps: 36%).
¹H-NMR (500 MHz, MeOD), δ 8.13 (d, 1H, ³J = 8.20 Hz, H-6), 5.91 (d, 1H, ³J = 6.00 Hz, H-1'), 5.79 (d, 1H, ³J = 8.20 Hz, H-5), 4.44 (dd, 1H, ³J = 5.05 Hz and ³J = 5.95 Hz, H-2'), 4.38 (d, 1H, ³J = 3.20 Hz, H-4'), 4.28 (dd, 1H, ³J = 5.05 Hz and ³J = 3.15 Hz, H-3'), 4.21-4.15 (2 x q, 4H, 2 x O-CH₂), 3.74 (d, 2H, ²J_{H,P} = 11.65 Hz, CH₂-P, methyl phosphonate), 3.51 (m, 2H, ³J = 6.65 Hz, N-CH₂, propaneamide), 2.53 (m, 2H, ³J = 6.60 Hz, O=C-CH₂, propaneamide), 1.37 (2 x t, 6H, 2 x CH₃).
¹³C-NMR (125 MHz, MeOD) δ 173.7 (C=O), 172.6 (C=O), 166.8 (C-4), 153.2 (C-2), 144.5 (C-6), 103.4 (C-5), 92.8 (C-1'), 85.4 (C-4'), 74.9 (C-2'), 74.0 (C-3'), 64.4 (2 x O-CH₂), 37.1 (N-CH₂, propaneamide), 36.4 (O=C-C_{H2}, propaneamide), 35.7 (d, ¹J_{C,P} = 145.7 Hz, CH₂-P, methyl phosphonate), 17.0 (2 x CH₃).
³¹P-NMR (202 MHz, MeaD) δ 24.6.
MS (ESI), m/z +1: 479.0; m/z -1: 477.1.

### G. 4-[(2S,3R,4S,5R)-5-(2,4-Dioxo-3,4-dihydropyrimidin-1(2H)-yl)-3,4-dihydroxy-tetrahydrofurane-2-carboxamidolbutaneamidomethylphosphonic acid diethylester (7)

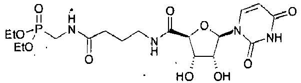

In a dry vessel, N-tert-butyloxycarbonyl-γ-aminobutyric acid (10 mmol, 2030' mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of aminomethylphosphonic acid diethyl ester oxalate (11 mmol, 2827 mg) in THF (10 ml) and 1N aq. NaOH (11 ml), pre-cooled on ice, was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. 3-Aminopropylcarboxamidomethylphosphonic acid diethyl ester hydrochloride was precipitated by addition of 50 ml of diethyl ether, filtered off and thoroughly washed with diethyl ether (yield over two steps: 2300 mg, 80%, white crystals).

Under an atmosphere of argon, 2',3'-anisylideneuridine-4'-carboxylic acid (1 mmol, 376 mg), PyBOP^{®} (1.1 mmol, 455 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, 3-aminopropylcarboxamidomethylphosphonic acid diethyl ester hydrochloride (2 mmol, 550 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneuridine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 310 mg of the title compound (7) was isolated by lyophilization as white amorphous powder (yield over two steps: 63%).
¹H-NMR (500 MHz, MeOD), δ 8.11 (d, 1H, ³J = 7.85 Hz, H-6), 5.84 (d, 1H, ³J = 6.00 Hz, H-1'), 5.78 (d, 1H, ³J = 8.20 Hz, H-5), 4.50 (dd, 1H, ³J = 5.05 Hz and ³J = 5.95 Hz, H-2'), 4.39 (d, 1H, ³J = 3.15 Hz, H-4'), 4.27 (dd, 1H, ³J = 5.05 Hz and ³J = 3.15 Hz, H-3'), 4.20-4.15 (2 x q, 4H, 2 x O-CH₂), 3.75 d, 2H, ²JH P = 11.65 Hz, CH₂-P, methyl phosphonate), 3.38-3.26 (m, partly below solvent peak, 2H, ³J = 6.95 Hz, N-CH₂, butaneamide), 2.34 (t, 2H, ³J = 7.55 Hz, O=C-CH₂, butaneamide), 1.88 (tt, 2H, ³J = 7.25 Hz and ³J = 6.95 Hz, CH₂, butaneamide), 1.36 (2 x t, 6H, 2 x CH₃).
¹³C-NMR (125 MHz, MeOD) δ 175.5 (C=O), 172.7 (C=O), 166.4 (C-4), 153.0 (C-2), 144.9 (C-6), 103.3 (C-5), 93.6 (C-1'), 85.5 (C-4'), 74.9 (C-2'), 73.9 (C-3'), 64.4 (2 x O-CH₂), 39.9 (N-CH₂, butaneamide), 35.6 (d, ¹J_{C,P} = 156.8 Hz, CH₂-P, methyl phosphonate), 34.3 (O=C-CH₂, butaneamide), 26.8 (CH₂, butaneamide), 17.0 (2 x CH₃).
³¹P-NMR (202 MHz, MeOD) δ 24.6.
MS (ESI), m/z +1: 493.1; m/z -1: 491.5.

### H. 2-[2-((2S,3R,4S,5R)-5-(2,4-Dioxo-3,4-dihydropyrimidin-1(2H)-yl)-3,4-dihydroxy-tetrahvdrofurane-2-carboxamido)ethaneamido]ethylphosphonic acid diethylester (8)

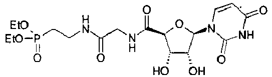

In a dry vessel, N-tert-butyloxycarbonylglycine (10 mmol, 1750 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of aminoethyl-phosphonic acid diethylester oxalate (11 mmol, 2981 mg) in THF (10 ml) and 1N, aq. NaOH (11 ml), pre-cooled on ice, was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. 2-(Aminomethylcarboxamido)ethylphosphonic acid diethyl ester hydrochloride was precipitated by addition of 50 ml of diethyl ether, filtered off and thoroughly washed with diethyl ether (yield over two steps: 1900 mg, 69%, clay). Under an atmosphere of argon, 2',3'-anisylideneuridine-4'-carboxylic acid (1 mmol, 376 mg), HCTU^{®} (1.1 mmol, 455 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, 2-(aminomethyl-carboxamido)ethylphosphonic acid diethyl ester hydrochloride (2 mmol, 550 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneuridine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 280 mg of the title compound (8) was isolated by lyophilization as white amorphous powder (yield over two steps 59%).
¹H-NMR (500 MHz, MeOD), δ 8.12 (d, 1H, ³J = 8.20 Hz, H-6), 5.93 (d, 1H, ³J = 5.95 Hz, H-1'), 5.78 (d, 1H, ³J = 8.20 Hz, H-5), 4.48 (dd, 1H, ³J = 5.35 Hz and ³J = 6.00 Hz, H-2'), 4.48 (d, 1H, ³J = 3.15 Hz, H-4'), 4.40 (dd, 1H, ³J = 5.05 Hz and ³J = 3.20 Hz, H-3'), 4.19 - 4.12 (2 x q, 4H, 2 x O-CH₂), 4.01 - 3.83 (AB-system with A d and B d, 2H, ²J_{A,B} = 16.70 Hz, N-CH₂, ethaneamide), 3.50 (m, 2H, ³J = 7.55 Hz and ³J_{H,P} = 12.95 Hz, N-CH₂, ethyl phosphonate), 2.16 - 2.09 (m, 2H, ³J = 7.55 and ²J_{H,P} = 18.35 Hz, CH₂-P, ethyl phosphonate), 1.37 (t, 6H, 2 x CH₃).
¹³C-NMR (125 MHz, MeOD) δ 173.2 (C=O), 171.6 (C=O), 166.4 (C-4), 153.0 (C-2), 144.6 (C-6), 103.4 (C-5), 93.1 (C-1'), 85.4 (C-4'), 74.8 (C-2'), 74.1 (C-3'), 63.8 (2 x O-CH₂), 43.5 (N-CH₂, ethaneamide), 34.9 (N-CH₂, ethyl phosphonate), 26.5 (d, ¹J_{C,P} = 138.3 Hz, CH₂-P, ethyl phosphonate), 17.0 (2 x CH₃).
³¹P-NMR (202 MHz, MeOD) δ 28.8.
MS (ESI), m/z +1: 479.0; m/z -1: 477.1.

### I. 2-[(2S,3R,4S,5R)-5-(2,4-Dioxo-3,4-dihydropyrimidin-1(2H)-yl)-3,4-dihydroxy-tetrahydro-furane-2-carboxamido]ethaneamido-ethyloxvcarbonylmethylenephos-phonic acid diethylester (9)

Commercially available N-benzyloxycarbonyl-α-phosphonoglycine (11 mmol, 3600 mg) was dissolved in 20 ml of dry methanol and hydrogenated for 1 hour at 3 atm H₂ (rt) with 1 g of Pd/C. The suspension was filtered, the catalyst washed with methanol (2 x 5 ml) and the filtrate directly used in the next step. In a dry vessel, N-tert-butyloxycarbonylglycine (10 mmol, 1750 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) the solution of α-phosphonoglycine in methanol (30 ml), pre-cooled on ice, was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. N-(Aminomethylcarbonyl)-α-dimethylphosphonoglycine methyl ester hydrochloride was precipitated by addition of 50 ml of diethyl ether, filtered off and thoroughly washed with diethyl ether (yield over two steps: 1650 mg, 65%, white crystals). Under an atmosphere of argon, 2',3'-anisylideneuridine-4'-carboxylic acid (1 mmol, 376 mg), HCTU^{®} (1.1 mmol, 455 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, N-(aminomethylcarbonyl)-α-dimethylphosphonoglycine methyl ester hydrochloride (2 mmol, 550 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneuridine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 260 mg of the title compound (9) was isolated as a mixture of two stereoisomeres by lyophilisation as white amorphous powder (yield over two steps: 53%).
¹H-NMR (500 MHz, MeOD), δ 8.10 (2 x d, 1H, ³J = 7.85 Hz, H-6), 5.96 (2 x d, 1H, ³J = 5.95 Hz, H-1'), 5.77 (2 x d, 1H, ³J = 7.85 Hz, H-5), 4.50-4.40 (m, 3H, H-2', H-3' and H-4'), 4.18-3.94 (AB-system with A d and B d, 2H, ²J = 17.00 Hz, N-CH₂ (ethaneamide), 3.97-3.85 (m, 9H, 3 x O-CH₃), N-CH (α-phosphonoglycine) not determinable, under solvent peak at 3.35 ppm.
¹³C-NMR (125 MHz, MeOD) δ 173.3 (C=O), 171.4 (C=O), 168.1 (C=O), 166.4 (C-4), 153.0 (C-2), 144.6 (C-6), 103.5 (C-5), 93.3 (C-1'), 85.6 (H-4'), 74.9 (H-2'), 74.2 (H-3'), 55.3 (3 x O-CH₃), N-CH (α-phosphonoglycine) not determinable, under solvent peak at 49 ppm, 43.1 (N-CH₂, ethaneamide)
³¹P-NMR (202 MHz, MeOD) δ 18.1.
MS (ESI), m/z +1: 495.0; m/z -1: 493.3

### J. 2-[(2S,3R,4S,5R)-5-(2,4-Dioxo-3,4-dihydropyrimidin-1(2H)-yl)-3,4-dihydroxy-tetrahydrofurane-2-carboxamidolethaneamidomethylenediphosphonic acid tetrae-thylester (10)

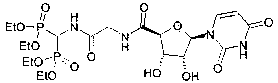

In a dry vessel, N-tert-butyloxycarbonyl-glycine (10 mmol, 1750 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of aminomethylenediphosphonic acid diethylester (11 mmol, 3350 mg) in dry THF (10 ml) was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. Aminomethylcarboxamidomethylbis-(phosphonic acid diethyl ester) hydrochloride was precipitated by addition of 50 ml of diethyl ether, filtered off and thoroughly washed with diethyl ether (yield over two steps: 3100 mg, 76%, clay).

Under an atmosphere of argon, 2',3'-anisylideneuridine-4'-carboxylic acid (1 mmol, 376 mg), HCTU^{®} (1.1 mmol, 455 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, aminomethylcarboxamidomethyl-bis(phosphonic acid diethyl ester) hydrochloride (2 mmol, 788 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneadenosine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 320 mg of the title compound (10) was isolated by lyophilization as white amorphous powder (yield over two steps: 50%).
¹H-NMR (500 MHz, DMSO-d₆), δ 11.30 (d, 1H, ³J = 1.85 Hz, NH, uracil), 8.74 (d, 1H, ³J = 9.80 Hz, CONH), 8.51 (t, 1H, ³J = 5.65 Hz, 4'-CONH), 8.23 (d, 1H, ³J = 8.15 Hz, H-6), 5.92 (d, 1H, ³J = 6.90 Hz, H-1'), 5.62 (dd, 1H, ³J = 7.90 Hz and ⁴J = 2.20 Hz, H-5), 5.52 (br s, 2H, 2 x OH), 4.82 (td, 1H, ³J = 9.75 Hz and ²J_{H,P} = 22.35 Hz, PPNCH, methylene diphosphonate), 4.35 (d, 1H, ³J = 1.90 Hz, H-4'), 4.20 - 3.99 (br s, 10H, 4 x O-CH₂, H-2'and H-3'), 3.85 (AB-system with A dd and B dd, 1H, ³J = 5.70 Hz and ²J = 17.30 Hz, N-CH₂, ethaneamide), 1.22 (br s, 12H, 4 x CH₃).
¹³C-NMR (125 MHz, DMSO-d₆) δ 170.5 (C=O), 168.7 (C=O), 163.2 (C-4), 151.2 (C-2), 141.4 (C-6), 102.2 (C-5), 87.8 (C-1'), 83.2 (C-4'), 73.9 (C-2'), 72.2 (C-3'), 63.1 (4 x O-CH₂), 43.5 (t, partially under solvent peak, ¹J_{C,P} = 581.90 Hz, PPNCH, methylene diphosphonate)), N-CH₂ (ethaneamide) under solvent peak at 42, 16.3 (4 × CH₃).
³¹P-NMR (202 MHz, DMSO-d₆) 15.8.

### K. 3-[(2S,3R,4S,5R)-5-(2,4-Dioxo-3,4-dihydropyrimidin-1(2H)-yl)-3,4-dihydroxy-tetrahydrofurane-2-carboxamido]propaneamidomethylenediphosphonic acid tetra-ethylester (11)

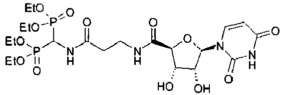

In a dry vessel, N-tert-butyloxycarbonyl-β-alanine (10 mmol, 1890 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of aminomethylenediphosphonic acid diethylester (11 mmol, 3350 mg) in dry THF (10 ml) was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. 2-Aminoethylcarboxamidomethyl-bis(phosphonic acid diethyl ester) hydrochloride was precipitated by addition of 50 ml of diethyl ether, filtered off and thoroughly washed with diethyl ether (yield over two steps: 3100 mg, 76%, clay).

Under an atmosphere of argon, 2',3'-anisylideneadenosine-4'-carboxylic acid (1 mmol, 376 mg), PyBOP^{®} (1.1 mmol, 572 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, 2-aminoethylcarboxamidomethyl-bis(phosphonic acid diethyl ester) hydrochloride (2 mmol, 816 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneadenosine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 320 mg of the title compound (11) was isolated by lyophilization as white amorphous powder (yield over two steps: 50%).
¹H-NMR (500 MHz, DMSO-d₆), δ 11.30 (d, 1H, ⁴J = 2.25 Hz, NH, uracil), 8.74 (d, 1H, ³J = 10.05 Hz, CONH, propaneamide), 8.28 (t, 1H, ³J = 5.65 Hz, 4'-CONH), 8.23 (d, 1H, ³J = 8.20 Hz, H-6), 5.88 (d, 1H, ³J = 6.30 Hz, H-1'), 5.69 (dd, 1H, ³J = 7.90 Hz and ⁴J = 2.20 Hz, H-5'), 5.48 (br s, 2H, 2'-OH and 3'-OH), 4.86 (dt, 1H, ³J = 10.1 Hz and ²J_{P,H} = 22.70 Hz, methylene diphosphonate), 4.23 (d, 1H, ³J = 2.20 Hz, H-4'), 4.16 (pseudo-t, 1H, ³J = 4.72 Hz and ³J = 6.60 Hz, H-2'), 4.02 (br s, 8H, 4 x O-CH₂), 3.99 (pseudo-q, 1H, ³J = 2.20 Hz and ³J = 2.20 Hz and ³J = 2.50 Hz, H-3'), 3.40 - 3.20 (N-CH₂, propaneamide), not determinable, covered by water from solvent), 2.44 (t, 2H, ³J = 6.95 Hz, O=C-CH₂, propaneamide), 1.21 (m, 12H, 4 x CH₃).
¹³C-NMR (125 MHz, DMSO-d₆), δ 172.1 (C=O), 170.2 (C=O), 163.2 (C-4), 151.1 (C-2), 143.5 (C-6), 102.2 (C-5), 88.0 (C-1'), 83.2 (C-4'), 73.1 (C-2'), 73.0 (C-3'), 63.0 and 62.8 (4 x O-CH₂), 43.3 (t, partially covered by solvent peak, ¹J = 579.90 Hz, PPNCH, methylene diphosphonate), 35.4 (N-CH₂ (propaneamide)), 34.4 (O=C-CH₂ propaneamide)), 16.4 and 16.3 (4 x CH₃).
³¹P-NMR (202 MHz, DMSO-d₆), 15.2.

### L. 4-[(2S,3R,4S,5R)-5-(2,4-Dioxo-3,4-dihydropyrimidin-1(2H)-yl)-3,4-dihydroxy-tetrahydrofurane-2-carboxamido]butaneamidomethylenediphosphonic acid tetraethylester (12)

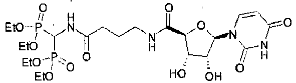

In a dry vessel, N-tert-butyloxycarbonyl-γ-aminobutyric acid (10 mmol, 2030 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of of aminomethylenediphosphonic acid diethylester (11 mmol, 3350 mg) in dry THF (10 ml) was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. 3-Aminopropylcarboxamidomethyl-bis(phosphonic acid diethyl ester) hydrochloride was precipitated by addition of 50 ml of diethyl ether, filtered off and thoroughly washed with diethyl ether (yield over two steps: 3400 mg, 80%, clay).

Under an atmosphere of argon, 2',3'-anisylideneuridine-4'-carboxylic acid (1 mmol, 376 mg), PyBOP^{®} (1.1 mmol, 572 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, 3-aminopropylcarboxamidomethyl-bis(phosphonic acid diethyl ester) hydrochloride (2 mmol, 844 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneadenosine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 380 mg of the title compound (12) was isolated by lyophilization as white amorphous powder (yield over two steps: 58%).
¹H-NMR (500 MHz, DMSO-d₆), δ 11.30 (d, 1H, ³J = 1.85 Hz, NH, uracil), 8.63 (d, 1H, ³J = 9.75. Hz, CONH), 8.31 (t, 1H, ³J = 5.65 Hz, 4'-CONH), 8.27 (d, 1H, ³J = 8.15 Hz, H-6), 5.86 (d, 1H, ³J = 6.30 Hz, H-1'), 5.62 (dd, 1H, ³J = 8.15 Hz and ⁴J = 2.20 Hz, H-5), 5.50 (br s, 2H, 2 x OH), 4.87 (td, 1H, ³J = 10.10 Hz and ²J_{H,P} = 23.00 Hz, methylenediphosphonate), 4.25 (d, 1H, ³J = 2.50 Hz, H-4'), 4.17 (pseudo-t, 1H, ³J = 4.75 Hz and ³J = 5.95 Hz, H-2'), 4.08 - 4.02 (m, 8H, 4 × O-CH₂), 3.98 (dd, 1H, ³J = 2.50 Hz and ³J = 4.40 Hz, H-3'), 3.08 (dt, 2H, ³J = 5.70 Hz and ³J = 7.50 Hz, N-CH₂, butaneamide), 2.23 (t, 2H, ³J = 7.25 Hz, O=C-CH₂, butaneamide), 1.65 (tt, 2H, ³J = 7.25 Hz, CH₂, butaneamide), 1.22 (br s, 12H, 4 x CH₃).
¹³C-NMR (125 MHz, DMSO-d₆) δ 171.8 (C=O), 170.0 (C=O), 163.2 (C-4), 151.2 (C-2), 141.4 (C-6), 102.1 (C-5), 88.3 (C-1'), 83.3 (C-4'), 73.2 (C-2'), 73.1 (C-3'), 62.9 (4 x O-CH₂), 43.5 (t, ¹J_{C,P} = 589.80 Hz, PPNCH, methylenediphosphonate), 38.3 (N-CH₂, butaneamide), 32.3 (O=C-CH₂, butaneamide), 25.5 (CH₂, butaneamide), 16.3 (4 x CH₃).
³¹P-NMR (202 MHz, DMSO-d₆) 15.6.

### M. 4-[2-((2S,3R,4S,5R)-5-(6-Amino-9H-purin-9-yl)-3,4-dihydroxy-tetrahydro-furane-2-carboxamido)ethaneamido]benzylphosphonic acid diethylester (CC 1) (reference)

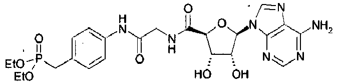

In a dry vessel, N-tert-butyloxycarbonylglycine (10 mmol, 1750 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of *p-*aminobenzylphosphonic acid diethyl ester (11 mmol, 2673 mg) in dry THF (10 ml) was added. The resulting mixture was allowed to warm to ambient temperature. After three hours, the volatiles were removed by rotary evaporation at 40 °C, the residue was dissolved in 10 ml of water and adjusted to pH 1 (10 % aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. *p*-(Aminomethylcarboxamido)benzylphosphonic acid diethyl ester hydrochloride was precipitated by addition of 50 ml of diethyl ether, filtered off and thoroughly washed with diethyl ether (yield over two steps: 3100 mg, 92%, white chrystals).

Under an atmosphere of argon, 2',3'-anisylideneadenosine-4'-carboxylic acid (1 mmol, 399 mg), HCTU^{®} (1.1 mmol, 455 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, p-(aminomethylcarboxamido)benzylphosphonic acid diethyl ester hydrochloride (2 mmol, 672 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane:methanol (40:1). The product was isolated by rotary evaporation at 40 °C and recrystallized from diethyl ether. Deprotection of the ribose moiety was performed by stirring 2',3'-anisylideneadenosine-5'-amide (100 mg) in a mixture of dichloromethane (3 ml), trifluoroacetic acid (0.15 ml) and water (0.1 ml) at ambient temperature. After two hours, the crude product was precipitated by the addition of diethyl ether (50 ml), filtered off, dissolved in 7 ml of water/methanol (75:25) and purified by RP-HPLC using a gradient of water/methanol from 75:25 to water/methanol 0:100. 310 mg of the title compound (CC 1) was isolated by lyophilization as white amorphous powder (yield over two steps: 68%).
¹H-NMR (500 MHz, MeOD), δ 8.42 (s, 1H, H-2), 8.24 (s, 1H, H-8), 7.57 (d, 2H, ³J = 8.20 Hz, 2 x CHₘₑₜₐ, benzyl phosphonate), 7.30 (dd, 2H, ³J = 8.80 Hz and ⁴J = 2.50 Hz, 2 x CHₒᵣₜₕₒ, benzyl phosphonate), 6.14 (d, 1H, ³J = 7.90 Hz, H-1'), 4.91 (dd, partly below solvent peak, 1H, ³J = 7.55 Hz and ³J = 4.75 Hz, H-2'), 4.62 (s, 1H, H-4'), 4.49 (dd, 1H, ³J = 4.75 and ³J = 1.60 Hz, H-3'), 4.29 - 4.10 (AB-system with A d and B d, 2H, ²J = 16.40 Hz, N-CH₂, ethaneamide), 4.09 - 4.04 (m, 4H, 2 × O-CH₂), 3.24 (d, 2H, ²J_{H,P} = 21.45 Hz, CH₂-P, benzyl phosphonate), 1.29 (t, 6H, 2 x CH₃).
¹³C-NMR (125 MHz, MeOD) δ 173.4 (C=O), 169.5 (C=O), 157.9 (C-6), 154.2 (C-2), 150.6 (C-4), 142.8 (C-8), 138.9 (C*ₚₐᵣₐ*, benzyl phosphonate), 131.7 (2 × CH*ₒᵣₜₕₒ*, benzyl phosphonate), 128.7 (d, ²J_{C,P} = 9.4 Hz, C*ᵢₚₛₒ*, benzyl phosphonate), 121.5 (2 x CH*ₘₑₜₐ*, benzyl phosphonate), 121.3 (C-5), 90.6 (C-1'), 86.7 (C-4'), 75.5 (C-2'), 73.9 (C-3'), 64.0 (2 x O-CH₂), 44.0 (N-CH₂, ethaneamide), 33.4 (d, ¹J_{C,P} = 137.6 Hz, CH₂-P, benzyl phosphonate), 17.0 und 16.9 (2 x CH₃).
³¹P-NMR (202 MHz, MeOD) δ 28.8.
MS (ESI), m/z +1: 564.3; m/z -1: 562.3.

### N. 4-[3-((2S,3R,4S,5R)-(6-Amino-9H-purin-9-yl)-3,4-dihydroxy-tetrahydro-furane-2-carboxamido)propaneamido]benzylphosphonic acid diethylester (CC 2) (reference)

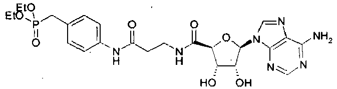

In a dry vessel, N-tert-butyloxycarbonyl-β-alanine (10 mmol, 1890 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of *p*-aminobenzylphosphonic acid diethyl ester (11 mmol, 2673 mg) in dry THF (10 ml) was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. *p*-(2-Aminoethylcarboxamido)benzylphosphonic acid diethyl ester hydrochloride was precipitated by addition of 50 ml of diethyl ether, filtered off and thoroughly washed with diethyl ether (yield over two steps: 3300 mg, 94%, white crystals).

Under an atmosphere of argon, 2',3'-anisylideneadenosine-4'-carboxylic acid (1 mmol, 399 mg), HBTU^{®} (1.1 mmol, 428 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, *p*-(2-aminoethylcarboxamido)benzylphosphonic acid diethyl ester hydrochloride (2 mmol, 700 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneadenosine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 350 mg of the title compound (CC 2) was isolated by lyophilization as white amorphous powder (yield over two steps: 71%).
¹H-NMR (500 MHz, D₂O), δ 8.08 (s, 1H, H-2), 7.95 (s, 1H, H-8), 7.57 (d, 2H, ³J = 8.20 Hz, 2 x CHₘₑₜₐ, benzyl phosphonate), 7.30 (dd, 2H, ³J = 8.80 Hz and ⁴J = 2.50 Hz, 2 x CHₒᵣₜₕₒ, benzyl phosphonate), 6.14 (d, 1H, ³J = 7.90 Hz, H-1'), 4.91 (dd, partially below solvent peak, 1H, ³J = 7.55 Hz and ³J = 4.75 Hz, H-2'), 4.62 (s, 1H, H-4'), 4.49 (dd, 1H, ³J = 4.75 and ³J = 1.60 Hz, H-3'), 4.09 - 4.04 (m, 4H, 2 × O-CH₂), 3.67 - 3.58 (m, 2H, ³J= 5.35 Hz, N-CH₂ (propaneamide), 3.24 (d, 2H, ²J_{H,P} = 21.45 Hz, CH₂-P, benzyl phosphonate), 2.59 - 2.49 (m, 2H, ³J_{X,A} = 5.35 Hz, O=C-CH₂, propaneamide), 1.29 (t, 6H, 2 x CH₃).
¹³C-NMR (125 MHz, D₂O) δ 174.9 (C=O), 174.4 (C=O), 157.9 (C-6), 155.3 (C-2), 150.7 (C-4), 143.9 (C-8), 138.8 (C*ₚₐᵣₐ*, benzyl phosphonate), 133.1 (2 × CH*ₒᵣₜₕₒ*, benzyl phosphonate), 129.6 (d, ²J_{C,P} = 9.4 Hz, Cᵢₚₛₒ, benzyl phosphonate), 123.1 (2 x CHₘₑₜₐ, benzyl phosphonate), 121.9 (C-5), 91.3 (C-1'), 87.4 (C-4'), 75.8 (C-2'), 74.7 (C-3'), 64.0 (2 x O-CH₂), 39.6 (N-CH₂, propaneamide), 39.1 (O=C-CH₂, propaneamide), .34.1 (d, ¹J_{C,P} = 137.6 Hz, CH₂-P, benzyl phosphonate), 18.3 (2 x CH₃).
³¹P-NMR (202 MHz, D₂O) δ 30.0.
MS (ESI), m/z +1: 578.2; m/z -1: 576.3.

### O. 4-[4-((2S,3R,4S,5R)-5-(6-Amino-9H-purin-9-yl)-3,4-dihydroxy-tetrahydro-furane-2-carboxamido)butaneamido]benzylphosphonic acid diethylester (CC 3) (reference)

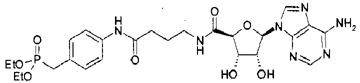

In a dry vessel, N-tert-butyloxycarbonyl-γ-aminobutyric acid (10 mmol, 2030 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. *p*-(3-Aminopropylcarboxamido) benzylphosphonic acid diethyl ester hydrochloride was precipitated by addition of 50 ml of diethyl ether, filtered off and thoroughly washed with diethyl ether (yield over two steps: 3300 mg, 94%, white crystals).

Under an atmosphere of argon, 2',3'-anisylideneadenosine-4'-carboxylic acid (1 mmol, 399 mg), HBTU^{®} (1.1 mmol, 428 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, p-(3-aminopropylcarboxamido)benzylphosphonic acid diethyl ester hydrochloride (2 mmol, 728 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneadenosine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 340 mg of the title compound (CC 3) was obtained by lyophilization as white amorphous powder (yield over two steps: 70%).
¹H-NMR (500 MHz, DMSO-d₆), δ 9.84 (br s, 1H, 4'-CONH), 9.05 (t, 2H, ³J = 5.70 Hz, CONH, butaneamide), 8.36 (s, 1H, H-2), 8.23 (s, 1H, H-8), 7.48 (d, 2H, ³J = 8.55 Hz, 2 x *CHₒᵣₜₕₒ,* benzyl phosphonate), 7.38 (br s, 2H, 6-NH₂), 7.16 (dd, 2H, ³J = 8.85 Hz and ⁴J = 2.20 Hz, 2 x CH*ₘₑₜₐ*, benzyl phosphonate), 5.95 (d, 1H, ³J = 7.60 Hz, H-1'), 5.72 (br d, 1H, ³J = 2.50 Hz, 2'-OH), 5.51 (br d, 1H, ³J = 4.40 Hz, 3'-OH), 4.61 (dd, 1H, ³J = 7.55 Hz and ³J = 4.75 Hz, H-2'), 4.32 (d, 1H, ³J = 1.60 Hz, H-4'), 4.14 (br s, 1H, H-3'), 3.95 - 3.89 (m, 4H, 2 x O-CH₂), 3.26 (dt, 2H, ³J = 7.25 Hz and ³J = 5.70 Hz, N-CH₂, butaneamide), 3.12 (d, 2H, ²J_{H,P} = 21.10 Hz, CH₂-P, benzyl phosphonate), 2.32 (t, 2H, ³J = 7.25 Hz, O=C-CH₂, butaneamide), 1.79 (tt, 2H, ³J = 7.25 Hz, CH₂, butaneamide), 1.29 (t, 6H, 2 x CH₃).
¹³C-NMR (125 MHz, DMSO-d₆) δ 170.8 (C=O), 169.6 (C=O), 156.5 (C-6), 152.6 (C-2), 148.9 (C-4), 140.9 (C-8), 137.9 *(Cₚₐᵣₐ,* benzyl phosphonate), 130.1 (2 × CH*ₒᵣₜₕₒ*, benzyl phosphonate), 126.7 (d, ²J_{C,P} = 9.4 Hz, C*ᵢₚₛₒ*, benzyl phosphonate), 119.8 (2 x CH*ₘₑₜₐ*, benzyl phosphonate), 119.1 (C-5), 88.0 (C-1'), 84.9 (C-4'), 73.4 (C-2'), 72.0 (C-3'), 61.5 (2 x O-CH₂), 38.5 (N-CH₂, butaneamide), 33.8 (O=C-CH₂, butaneamide), 33.4 (d, ¹J_{C,P} = 137.6 Hz, CH₂-P, benzyl phosphonate), 25.3 (CH₂, butaneamide), 16.4 und 16.3 (2 x CH₃).
³¹P-NMR (202 MHz, DMSO-d₆) δ 27.1.
m/z +1: 592.0; m/z -1: 590.3.

### P. 3-[(2S,3R,4S,5R)-5-(6-Amino-9H-purin-9-yl)-3,4-dihydroxy-tetrahydrofurane-2-carboxamido]propaneamidomethylenediphosphonic acid tetraethylester (CC 4) (reference)

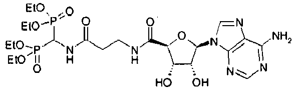

In a dry vessel, N-tert-butyloxycarbonyl-β-alanine (10 mmol, 1890 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of aminomethylenediphosphonic acid diethylester (11 mmol, 3350 mg) in dry THF (10 ml) was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. 2-Aminoethylcarboxamidomethyl-bis(phosphonic acid diethyl ester) hydrochloride was precipitated by addition of 50 ml of diethyl ether, filtered off and thoroughly washed with diethyl ether (yield over two steps: 3100 mg, 76%, clay).

Under an atmosphere of argon, 2',3'-anisylideneadenosine-4'-carboxylic acid (1 mmol, 399 mg), PyBOP^{®} (1.1 mmol, 572 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, 2-aminoethylcarboxamidomethyl-bis(phosphonic acid diethyl ester) hydrochloride (2 mmol, 816 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneadenosine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0: 100. 320 mg of the title compound (CC 4) was isolated by lyophilization as white amorphous powder (yield over two steps: 50%).
¹H-NMR (500 MHz, MeOD), δ 8.41 (s, 1H, H-2), 8.38 (s, 1H, H-8), 6.06 (d, 1H, ³J = 7.90 Hz, H-1'), 5.10 (t, 1H, ²J_{H,P} = 23.00 Hz, PPNCH, methylene diphosphonate), 4.77 (pseudo-q, 1H, ³J = 7.55 Hz and ³J = 4.75 Hz, H-2'), 4.51 (d, 1H, ³J = 1.55 Hz, H-4'), 4.36 (dd, 1H, ³J = 5.05 Hz and ³J = 1.25 Hz, H-3'), 4.23 - 4.07 (m, 8H, 4 x O-CH₂), 3.74 - 3.61 (m, 2H, ³J = 6.30 Hz, N-CH₂, propaneamide), 2.66 - 2.61 (m, 2H, ³J = 6.30 Hz, O=C-CH₂, propaneamide), 1.36 - 1.25 (m, 12H, 4 x CH₃).
¹³C-NMR (125 MHz, MeOD) δ 173.1 (C=O), 172.8 (C=O), 157.9 (C-6), 154.1 (C-2), 150.5 (C-4), 142.8 (C-8), 121.5 (C-5), 90.7 (C-1'), 86.8 (C-4'), 75.3 (C-2'), 73.7 (C-3'), 65.4 (4 x O-CH₂), 47.5 (t, ¹J_{C,P} = 579.9 Hz, PPNCH, methylene diphosphonate), 36.7 (N-CH₂, propaneamide), 36.2 (O=C-CH₂, propaneamide), 16.9 (4 × CH₃).
³¹P-NMR (202 MHz, MeOD) δ 15.5.
MS (ESI), m/z +1: 638.0; m/z -1: 636.3.

### Q. 4-[(2S,3R,4S,5R)-5-(6-Amino-9H-purin-9-yl)-3,4-dihydroxy-tetrahydrofurane-2-carboxamido]butaneamidomethylenediphosphonic acid tetraethylester (CC 5) (reference)

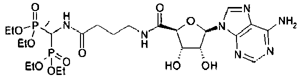

In a dry vessel, N-tert-butyloxycarbonyl-γ-aminobutyric acid (10 mmol, 2030 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of aminomethylenediphosphonic acid diethylester (11 mmol, 3350 mg) in dry THF (10 ml) was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 m of dry 4N HCl-dioxane solution and stirred for two hours at ambient temperature. 3-Aminopropylcarboxamidomethyl-bis(phosphonic acid diethyl ester) hydrochloride was precipitated by addition of 50 ml of diethyl ether, filtered off and thoroughly washed with diethyl ether (yield over two steps: 3400 mg, 80%, clay).

Under an atmosphere of argon, 2',3'-anisylideneadenosine-4'-carboxylic acid (1 mmol, 399 mg), PyBOP^{®} (1.1 mmol, 572 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, 3-aminopropylcarboxamidomethyl-bis(phosphonic acid diethylester) hydrochloride (2 mmol, 844 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneadenosine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 380 mg of the title compound (CC 5) was isolated by lyophilization as white amorphous powder (yield over two steps: 58%).
¹H-NMR (500 MHz, MeOD), δ 8.36 (s, 1H, H-2), 8.34 (s, 1H, H-8), 6.07 (d, 1H, ³J = 7.85 Hz, H-1'), 5.14 (t, 1H, ²J_{H,P} = 23.00 Hz, PPNCH, methylenediphosphonate), 4.79 (dd, 1H, ³J = 7.55 Hz and ³J = 4.70 Hz, H-2'), 4.51 (d, 1H, ³J = 1.25 Hz, H-4'), 4.36 (dd, 1H, ³J = 4.70 Hz and ³J = 1.25 Hz, H-3'), 4.26 - 4.19 (m, 8H, 4 × O-CH₂), 3.42 (t, 2H, ³J = 6.95 Hz, N-CH₂, butaneamide), 2.41 (t, 2H, ³J = 7.25 Hz, O=C-CH₂, butaneamide), 1.94 (tt, 2H, ³J = 7.25 Hz and ³J = 6.90 Hz, CH₂, butaneamide), 1.36 - 1.25 (m, 12H, 4 × CH₃).
¹³C-NMR (125 MHz, MeOD), δ 175.0 (C=O), 172.7 (C=O), 158.0 (C-6), 154.3 (C-2), 150.5 (C-4), 143.0 (C-8), 121.5 (C-5), 90.9 (C-1'), 86.8 (C-4'), 75.3 (C-2'), 73.7 (C-3'), 65.3 (4 x O-CH₂), 45.0 (t, ¹J_{C,P} = 596.8 Hz, PPNCH, methylenediphosphonate), 39.9 (N-CH₂, butaneamide), 34.0 (O=C-CH₂, butaneamide), 27.3 (CH₂, butaneamide), 17.0 (4 x CH₃).
³¹P-NMR (202 MHz, MeOD), δ 15.8.
MS (ESI), m/z +1: 652.3; m/z -1: 650.3.

### R. 2-[3-((2S,3R,4S,5R)-5-(2,4-Dioxo-3,4,5,6-tetrahydropyrimidin-1(2H)-yl)-3,4-dihydroxytetrahydrofurane-2-carboxamido)propaneamido]-(S)-aspartic acid (13)

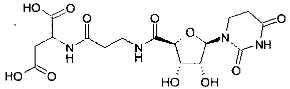

In a dry vessel, N-tert-butyloxycarbonyl-β-alanine (10 mmol, 1890 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of (*S*)-aspartic acid dibenzyl ester p-toluene sulfonate (11 mmol, 5330 mg) in 1N aq. NaOH-solution (11 ml) was added. The resulting mixture was allowed to warm to ambient temperature. After three hours, THF and other volatiles were removed by rotary evaporation at 40 °C, the residual aqueous mixture was diluted with a small volume of H₂O and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. (*S*)-2-Aminopropionylaspartic acid dibenzyl ester hydrochloride was precipitated by addition of 50 ml of diethyl ether, filtered off and thoroughly washed with diethyl ether (yield over two steps: 2600 mg, 68%, clay). Under an atmosphere of argon, 2',3'-anisylideneuridine-4'-carboxylic acid (1 mmol, 376 mg), PyBOP^{®} (1.1 mmol, 572 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, 2-aminoethylcarboxamidoglutamic acid dibenzyl ester hydrochloride (2 mmol, 840 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneuridine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 2-[3-((*2S*,*3R*,*4S*,*5R*)-5-(2,4-Dioxo-3,4,5,6-tetrahydropyrimidin-1(*2H*)-yl)-3,4-dihydroxytetrahydrofurane-2-carboxamido)propaneamido]-(*S*)-aspartic acid dibenzylester was isolated by lyophilisation (yield over two steps: 200 mg, 32%, white amorphous powder). The dibenzylester (30 mg, 0.05 mmol) was suspended by sonification in 2 ml of MeOH and water (5:1). Then, the catalyst Pd(OH)₂ (5 mg) was added, the vessel was purged first by argon and then by hydrogen which were applied by means of a balloon. The reaction was performed overnight at ambient temperature and checked by TLC. After 12 hours the catalyst was filtered off and thoroughly washed with methanol and water. The washings were added to the filtrate. The solvent was removed by lyophilisation to give 19 mg analytically pure title compound (13) as white amorphous powder (yield: 89%).
¹H-NMR (500 MHz, D₂O), δ 5.84 (d, 1H, ³J = 6.35 Hz, H-1'), 4.71 (t, 1H, ³J = 5.35 Hz, N-CH, Asp), 4.37 (d, 1H, ³J = 2.55 Hz, H-4'), 4.35 (dd, 1H, ³J = 5.35 Hz and ³J = 6.00 Hz, H-2'), 4.33 (dd, 1H, ³J = 2.50 Hz and ³J = 5.35 Hz, H-3'), 3.73 (m, 2H, ³J = 6.60 Hz, N-CH₂, dihydrouracil), 3.52 and 3.21 (AB-system with A dd and B dd, 2H, ³J= 6.65 Hz and ²J = 14.80 Hz, O=C-CH₂, Asp), 2.91 (t, 2H, ³J = 6.30 Hz, N-CH₂, propaneamide), 2.79 (m, 2H, ³J = 6.30 Hz and ³J = 2.85 Hz, O=C-CH₂, dihydrouracil), 2.56 (t, 2H, ³J = 6.30 Hz, O=C-CH₂, propaneamide).
¹³C-NMR (125 MHz, D₂O), δ 177.6 (C=O), 176.6 (2 x C=O), 176.4 (C=O), 174.3 (C-4), 157.6 (C-2), 91.5 (C-1'), 84.8 (C-4'), 75.4 (C-2'), 72.3 (C-3'), 45.4 (N-CH, Asp), 40.6 (N-CH₂, dihydrouracil), 39.0 (N-CH₂, propaneamide), 38.4 (O=C-CH₂, dihydrouracil), 37.5 (O=C-CH₂, Asp), 33.0 (O=C-CH₂, propaneamide).

### S. 2-[3-((2S,3R,4S,5R)-5-(2,4-Dioxo-3,4,5,6-tetrahydropyrimidin-1(2H)-yl)-3,4-dihydroxy-tetrahydrofurane-2-carboxamido)propaneamido]-(S)-glutamic acid (14)

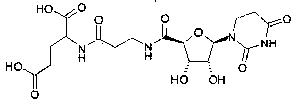

In a dry vessel, N-tert-butyloxycarbonyl-β-alanine (10 mmol, 1890 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of (S)-glutamic acid dibenzyl ester hydrochloride (11 mmol, 3883 mg) in 1N aq. NaOH-solution (11 ml) was added. The resulting mixture was allowed to warm to ambient temperature. After three hours, THF and other volatiles were removed by rotary evaporation at 40 °C, the residual aqueous mixture was diluted with a small volume of H₂O and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. (S)-2-Aminopropionylglutamic acid dibenzyl ester hydrochloride was precipitated by addition of 50 ml of diethyl ether, filtered off and thoroughly washed with diethyl ether (yield over two steps: 2800 mg, 65%, clay). Under an atmosphere of argon, 2',3'-anisylideneuridine-4'-carboxylic acid (1 mmol, 376 mg), PyBOP^{®} (1.1 mmol, 572 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, 2-aminoethylcarboxamidoglutamic acid dibenzyl ester hydrochloride (2 mmol, 868 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneuridine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 2-[3-((*2S*,*3R*,*4S*,*5R*)-5-(2,4-Dioxo-3,4,5,6-tetrahydropyrimidin-1(*2H*)-yl)-3,4-dihydroxytetrahydrofurane-2-carboxamido)propaneamido]-(*S*)-glutamic acid dibenzylester was isolated by lyophilisation (yield over two steps: 170 mg, 36%, white amorphous powder).

The dibenzylester (30 mg, 0.05 mmol) was suspended by sonification in 2 ml of MeOH and water (5:1). Then, the catalyst Pd(OH)₂ (5 mg) was added, the vessel was purged first by argon and then by hydrogen which were applied by means of a balloon. The reaction was performed overnight at ambient temperature and checked by TLC. After 12 hours the catalyst was filtered off and thoroughly washed with methanol and water. The washings were added to the filtrate. The solvent was removed by lyophilisation to give 20 mg analytically pure title compound (14) as white amorphous powder (yield: 90%).
¹H-NMR (500 MHz, D₂O), δ 5.84 (d, 1H, ³J = 6.30 Hz, H-1'), 4.37 (t, 1H, ³J = 6.00 Hz, N-CH, Glu),4.38 (d, 1H, ³J = 2.50 Hz, H-4'), 4.35 (dd, 1H, ³J = 5.35 Hz and ³J = 6.35 Hz, H-2'), 4.32 (dd, 1H, ³J = 2.20 Hz and ³J = 5.35 Hz, H-3'), 3.74 (m, 2H, ³J = 6.60 Hz, N-CH₂, dihydrouracil), 3.52 (t, 2H, ³J = 6.30 Hz, N-CH₂, propaneamide), 2.80 (m, 2H, ³J = 6.30 Hz and ³J = 2.85 Hz, O=C-CH₂, dihydrouracil), 2.56 (t, 2H, ³J = 6.30 Hz, O=C-CH₂, Glu), 2.45 (t, 2H, ³J = 7.55 Hz, O=C-CH₂, propaneamide), 2.15 (m, 1H, 0.5 x CH₂, Glu), 1.96 (m, 1H, 0.5 x CH₂, Glu).
¹³C-NMR (125 MHz, D₂O), δ 180.5 (C=O), 179.5 (C=O), 176.6 (C=O), 176.4 (C=O), 174.3 (C-4), 157.6 (C-2), 91.5 (C-1'), 84.8 (C-4'), 75.4 (C-2'), 72.3 (C-3'), 51.7 (N-CH, Glu), 45.4 (N-CH₂, dihydrouracil), 40.6 (O=C-CH₂, dihydrouracil), 38.4 (N-CH₂, propaneamide) 37.6 (O=C-CH₂, propaneamide), 33.4 (O=C-CH₂, Glu), 29.4 (CH₂, Glu).

### T. 2-[4-((2S,3R,4S,5R)-5-(2,4-Dioxo-3,4-dihydropyrimidin-1(2H)-yl)-3,4-dihydroxy-tetrahydrofurane-2-carboxamido)butaneamido]-(S)-aspartic acid-1-ethylester (15)

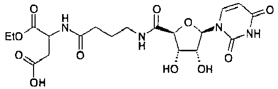

In a dry vessel, N-tert-butyloxycarbonyl-β-alanine (10 mmol, 1890 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of aspartic acid diethyl ester hydrochloride (11 mmol, 2475 mg) in 1N aq. NaOH-solution (11 ml) was added. The resulting mixture was allowed to warm to ambient temperature. After three hours, THF and other volatiles were removed by rotary evaporation at 40 °C, the residual aqueous mixture was diluted with a small volume of H₂O and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. (S)-2-Aminopropionylaspartic acid diethyl ester hydrochloride was precipitated by addition of 50 ml of diethyl ether, filtered off and thoroughly washed with diethyl ether (yield over two steps: 2800 mg, 65%, clay). Under an atmosphere of argon, 2',3'-anisyliderieuridine-4'-carboxylic acid (1 mmol, 376 mg), PyBOP^{®} (1.1 mmol, 572 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, (S)-2-aminoethylcarbonylaspartic acid dibenzyl ester hydrochloride (2 mmol, 550 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuum at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneuridine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 2-[4-((2*S*,3*R*,4*S*,5*R*)-5-(2,4-Dioxo-3,4-dihydropyrimidin-1(2*H*)-yl)-3,4-dihydroxy-tetrahydrofurane-2-carboxamido)butaneamido]-(*S*)-aspartic acid diethylester was isolated by lyophilisation (yield over two steps: 250 mg, 49%).
2-[4-((2*S*,3*R*,4*S*,5*R*)-5-(2,4-Dioxo-3,4-dihydropyrimidin-1(2*H*)-yl)-3,4-dihydroxy-tetrahydro-furane-2-carboxamido)butaneamido]-(*S*)-aspartic acid diethylester was dissolved in 20 ml of aq. Na₂HPO₄-bufferedsolution (50 mM buffer, pH 7.4) at 37°C. Pig liver esterase (20 mg) was added and the slightly cloudy solution was stirred overnight at 37 °C, filtered and lyophilisated. The lyophilisate was dissolved in 7 ml of water/methanol (90:10) and purified by RP-HPLC using a gradient of water/methanol from 90:10 to water/methanol 0:100. The solvents contain 0.1 % of trifuoracetic acid. 8 mg of the title compound (15) was isolated by lyophilization as white amorphous powder (yield: 42%).
¹H-NMR (500 MHz, D₂O), δ 7.97 (d, 1H, ³J = 8.20 Hz, H-6), 5.91 (d, 1H, ³J = ₇.90 Hz, H-5), 5.88 (d, 1H, ³J = 5.35 Hz, H-1'), 4.78 (t, 1H, ³J = 6.95 Hz, N-CH, Asp), 4.51 (pseudo-t, 1H, ³J = 5.35 Hz and ³J = 5.05 Hz, H-2'), 4.46 (d, 1H, ³J = 4.70 Hz, H-4'), 4.42 (pseudo-t, 1H, ³J = 5.00 Hz and ³J = 4.75 Hz, H-3'), 4.18 (q, 2H, O-CH₂), 3.28 (dt, 2H, ³J = 6.60 Hz and ³J = 6.95 Hz, N-CH₂, butaneamide), 2.91 (AB-system with A dd and B dd, 2H, ³J = 5.35 Hz and ²J = 16.70 Hz, O=C-CH₂, Asp), 2.34 (t, 2H, ³J = 6.95 Hz, O=C-CH₂, butaneamide), 1.83 (dt, 2H, ³J = 7.25 Hz and ³J = 6.95 Hz, CH₂, butaneamide), 1.27 (t, 3H, CH₃).
¹³C-NMR (125 MHz, D₂O), δ 178.6 (C=O), 176.8 (C=O), 175.4 (C=O), 174.2 (C=O), 169.0 (C-4), 154.5 (C-2), 146.1 (C-6), 105.2 (C-5), 94.3 (C-1'), 85.6 (C-4'), 73.4 (C-2'), 73.1 (C-3'), 65.1 (O-CH₂), 51.9 (N-CH, Asp), 41.3 (N-CH₂, butaneamide), 38.8 (O=C-CH₂, Asp), 35.5 (O=C-CH₂, butaneamide), 27.4 (CH₂, butaneamide), 16.1 (CH₃).

### U. 4-[2-((2S,3R,4S,5R)-5-(2,4-Dioxo-2,4-dihydro-3-methylpyrimidine-1-yl)-3,4-di-hydroxy-tetrahydrofurane-2-carboxamido)ethaneamido]benzylphosphonic acid diethylester (16)

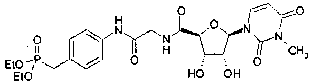

In a dry vessel, N-tert-butyloxycarbonylglycine (10 mmol, 1750 mg) was dissolved in 10 ml of dry THF and the solution was cooled to -25 °C. N-methylmorpholine (10 mmol, 1010 mg) and subsequently isobutyl chloroformate (10 mmol, 1360 mg) was added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) was added. The resulting mixture was allowed to warm to rt. After 3 h, the volatiles were removed by rotary evaporation at 40 °C. The residue was dissolved in 10 ml of water and adjusted to pH 1 (10 % aq. NaHSO₄ solution) and extracted with ethyl acetate (3 x 50 ml). The combined organic layers were washed with a saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for 2 h at rt. p-(Aminomethylcarboxamido)benzylphosphonic acid diethyl ester hydrochloride is precipitated by the addition of 50 ml of diethyl ether, filtered off and thoroughly washed with diethyl ether (yield over two steps: 3100 mg, 92%, white crystals). Under an atmosphere of argon, 2',3'-anisylidene-3-methyluridine-4'-carboxylic acid (1 mmol, 390 mg), HCTU^{®} (1.1 mmol, 455 mg) and 1-hydroxybenzotriazole (1.1 mmol, 149 mg) were dissolved in 2 ml of dry DMF at rt. Diisopropylethylamine (1.1 mmol, 143 mg) and, after 1 min, p-(aminomethylcarboxamido)benzylphosphonic acid diethyl ester hydrochloride (2 mmol, 672 mg), dissolved in a mixture of dry DMF (2 ml) and diisopropylethylamine (0.5 ml), are added sequentially via a syringe to the solution. Vigorous stirring was continued for 24 h at rt. The volatiles are removed in vacuum at 40 °C and the residue was purified by silica gel column chromatography using dichloromethane : methanol (40 : 1). The product was isolated by rotary evaporation at 40 °C and recrystallized from diethyl ether. Deprotection of the ribose moiety was performed by stirring 2',3'-anisylidene-3-methyluridine-5'-amide (100 mg) in a mixture of dichloromethane (3 ml), trifluoroacetic acid (0.15 ml) and water (one drop) at rt. After 2 h, the crude product was precipitated by the addition of diethyl ether (50 ml), filtered off, dissolved in 7 ml of water : methanol (75 : 25) and purified by RP-HPLC using a gradient of water : methanol from 75 : 25 to water : methanol 0 · 100. 340 mg of the title compound (16) was obtained by lyophilization as white amorphous powder (yield over two steps: 61%).
¹H-NMR (500 MHz, MeOD), δ 8.18 (d, 1H, ³J = 7.90 Hz, H-6), 7.58 (d, 2H, ³J = 8.20 Hz, 2 x CHₘₑₜₐ, benzylphosphonate), 7.31 (dd, 2H, ³J = 8.50 Hz and ⁴J = 2.50 Hz, 2 x CHₒᵣₜₕₒ, benzylphosphonate), 6.02 (d, 1H, ³J = 5.65 Hz, H-1'), 5.84 (d, 1H, ³J = 8.20 Hz, H-5), 4.50 (d, 1H, ³J = 3.15 Hz, H-4'), 4.46 (dd, 1H, ³J = 5.05 Hz and ³J = 5.65 Hz, H-2'), 4.41 (dd, 1H, ³J = 3.15 Hz and ³J = 5.05 Hz, H-3'), 4.18 - 3.99 (AB-system with A d and B d, partially overlapping with 2 x O-CH₂, 2H, ²J = 16.35 Hz, N-CH₂, 2-amidoethanamide), 4.09 - 4.01 (2 x q, 4H, 2 x O-CH₂), 3.29 (s, 3H, 3-CH₃), 3.25 (d, 2H, ²J_{H,P} = 21.45 Hz, CH₂-P, benzylphosphonate), 1.27 (t, 6H, 2 x CH₃).
¹³C-NMR (125 MHz, MeOD), δ 173.2 (C=O), 169.5 (C=O), 165.4 (C-4), 153.1 (C-2), 142.3 (C-6), 138.8 (C-NH, benzylphosphonate), 131.7 (2 × CHₘₑₜₐ, benzylphosphonate), 128.8 (d, ²J_{C,P} = 9.4 Hz, C-CH₂-P, benzylphosphonate), 121.5 (2 x CHₒᵣₜₕₒ, benzylphosphonate), 102.6 (C-5), 93.6 (C-1'), 85.3 (C-4), 74.9 (C-2), 74.4 (C-3), 64.0 (2 x O-CH₂), 43.9 (N-CH₂, 2-amidoethaneamide), 33.4 (d, ¹J_{C,P} = 137.5 Hz, CH₂-P, benzylphosphonate), 28.4 (3-CH₃), 17.0 and 16.9 (2 x CH₃).
³¹P-NMR (202 MHz, MeOD), δ 26.7.

### V. 4-12-((2S,3R,4S,5R)-5-(2,4-Dioxo-2,4-dihydro-3-ethylpyrimidine-1-yl)-3,4-dihydroxy-tetrahydrofurane-2-carboxamido)ethaneamidolbenzylphosahonic acid diethylester (17)

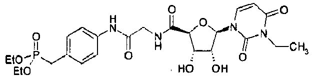

Under an athmosphere of argon, 2',3'-anisylidene-3-ethyluridine-4'-carboxylic acid (1 mmol, 404 mg), HCTU^{®} (1.1 mmol, 455 mg) and 1-hydroxybenzotriazole (1.1 mmol, 149 mg) were dissolved in 2 ml of dry DMF at rt. Diisopropylethylamine (1.1 mmol, 143 mg) and, after one min, p-(aminomethylcarboxamido)benzylphosphonic acid diethyl ester hydrochloride (2 mmol, 672 mg), dissolved in a mixture of dry DMF (2 ml) and diisopropylethylamine (0.5 ml), were added via a syringe to the solution. Vigorous stirring was continued for 24 h at rt. The volatiles were removed in vacuum at 40 °C and the residue is purified by silica gel column chromatography using dichloromethane:methanol (40 : 1). The product was isolated by rotary evaporation at 40 °C and recrystallized from diethyl ether. Deprotection of the ribose moiety was performed by stirring 2',3'-anisylidene-3-ethyluridine-5'-amide (100 mg) in a mixture of dichloromethane (3 ml), trifluoroacetic acid (0.15 ml) and water (one drop) at rt. After 2 h, the crude product was precipitated by the addition of diethyl ether (50 ml), filtered off, dissolved in 7 ml of water : methanol (75 : 25) and purified by RP-HPLC using a gradient of water : methanol from 75 : 25 to water : methanol 0 : 100. 370 mg of the title compound (17) was obtained by lyophilization as white amorphous powder (yield over two steps: 63%).
¹H-NMR (500 MHz, DMSO-d₆), δ 9.98 (s, 1H, CONH); 8.57 (t, 1H, ³J = 6.00 Hz, 4'-CONH), 8.30 (d, 1H, ³J = 8.20 Hz, H-6), 7.20 (d, 2H, ³J = 8.20 Hz, 2 x CHₘₑₜₐ, benzylphosphonate), 7.31 (dd, 2H, ³J = 8.50 Hz and ⁴J = 2.50 Hz, 2 x CHₒᵣₜₕₒ, benzylphosphonate), 5.99 (d, 1H, ³J = 6.60 Hz, H-1'), 5.78 (d, 1H, ³J = 8.20 Hz, H-5), 5.56 (d, 1H, ³J = 4.75 Hz, 3'-OH), 5.54 (d, 1H, ³J = 6.00 Hz, 2'-OH), 4.40 (d, 1H, ³J = 2.20 Hz, H-4'), 4.46 (pseudo-q, 1H, ³J = 4.75 Hz and ³J = 5.95 Hz and ³J = 6.00 Hz, H-2'), 4.41 (ddd, 1H, ³J = 2.20 Hz and ³J = 4.75 Hz, H-3'), 3.96 - 3.85 (AB-system with A d and B d, overlapping with 2 x O-CH₂, 2H, N-CH₂, 2-amidoethanamide), 3.93 - 3.91 (2 x q, 4H, 2 x O-CH₂), 3.83 (q, 2H, ³J = 6.90 Hz, 3-CH₂), 3.14 (d, 2H, ²J_{H,P} = 21.45 Hz, CH₂-P, benzylphosphonate), 1.15 (t, 6H, 2 x CH₃), 1.08 (t, 3H, ³J = 6.95 Hz, 3-CH₂-CH₃).
¹³C-NMR (125 MHz, DMSO-d₆), δ 170.6 (C=O), 167.3 (C=O), 161.8 (C-4), 151.0 (C-2), 139.9 (C-6), 137.4 (C-NH, benzylphosphonate), 130.2 (2 x CHₘₑₜₐ, benzylphosphonate), 127.2 (d, ²J_{C,P} = 9.4 Hz, C-CH₂-P, benzylphosphonate); 119.2 (2 x CHₒᵣₜₕₒ, benzylphosphonate), 101.5 (C-5), 88.9 (C-1'), 83.2 (C-4), 73.2 (C-2), 73.1. (C-3), 61.5 (2 x O-CH₂), 42.6 (N-CH₂, 2-amidoethaneamide), 35.6 (3-CH₂), 31.8 (d, ¹J_{C,P} = 137.5 Hz, CH₂-P, benzylphosphonate), 16.3 (2 x CH₃), 12.8 (3-CH₂-CH₃).
³¹P-NMR (202 MHz, DMSO-d₆), δ 27.2.

### W. 4-[2-((2S,3R,4S,5R)-5-(2,4-Dioxo-2,4-dihydro-3-butylpyrimidine-1-yl)-3,4-dihydroxy-tetrahydrofurane-2-carboxamido)ethaneamidolbenzylphosahonic acid diethylester (18)

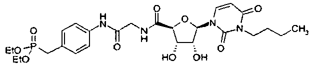

In a dry vessel, N-tert-butyloxycarbonylglycine (10 mmol, 1750 mg) was dissolved in 10 ml of dry THF and cooled to -25 °C. Subsequently, N-methylmorpholine (10 mmol, 1010 mg) and isobutyl chloroformate (10 mmol, 1360 mg) were added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) was added. The resulting mixture was allowed to warm to rt. After 3 h, the volatiles were removed by rotary evaporation at 40 °C, the residue was dissolved in 10 ml of water and adjusted to pH 1 (10 % aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for 2 h at rt. p-(Aminomethylcarboxamido)benzylphosphonic acid diethyl ester hydrochloride was precipitated by the addition of 50 ml of diethyl ether, filtered off and thoroughly washed with diethyl ether (yield over two steps: 3100 mg, 92%, white crystals).

Under an atmosphere of argon, 2',3'-anisylidene-3-butyluridine-4'-carboxylic acid (1 mmol, 432 mg), HCTU^{®} (1.1 mmol, 455 mg) and 1-hydroxybenzotriazole (1.1 mmol, 149 mg) were dissolved in 2 ml of dry DMF at rt. Diisopropylethylamine (1.1 mmol, 143 mg) and, after 1 min, p-(aminomethylcarboxamido)benzylphosphonic acid diethyl ester hydrochloride (2 mmol, 672 mg), dissolved in a mixture of dry DMF (2 ml) and diisopropylethylamine (0.5 ml), were added via a syringe to the solution. Vigorous stirring was continued for 24 h at rt. The volatiles were removed in vacuum at 40 °C and the residue is purified by silica gel column chromatography using dichloromethane : methanol (40 : 1). The product was isolated by rotary evaporation at 40 °C and recrystallized from diethyl ether. Deprotection of the ribose moiety was performed by stirring 2',3'-anisylideneuridine-5'-amide (100 mg) in a mixture of dichloromethane (3 ml), trifluoroacetic acid (0.15 ml) and water (one drop) at rt. After 2 h, the crude product was precipitated by the addition of diethyl ether (50 ml), filtered off, dissolved in 7 ml of water : methanol (75 : 25) and purified by RP-HPLC using a gradient of water : methanol from 75 : 25 to water : methanol 0 : 100. 420 mg of the title compound (18) was obtained by lyophilization as white amorphous powder (yield over two steps: 70%).
¹H-NMR (500 MHz, MeOD), δ 8.18 (d, 1H, ³J = 7.90 Hz, H-6), 7.58 (d, 2H, ³J = 8.20 Hz, 2 x CHₘₑₜₐ, benzylphosphonate), 7.31 (dd, 2H, ³J = 8.50 Hz and ⁴J = 2.50 Hz, 2 x CHₒᵣₜₕₒ, benzylphosphonate), 6.02 (d, 1H, ³J = 5.65 Hz, H-1'), 5.84 (d, 1H, ³J = 8.20 Hz, H-5), 4.50 (d, 1H, ³J = 3.15 Hz, H-4'), 4.46 (dd, 1H, ³J = 5.05 Hz and ³J = 5.65 Hz, H-2'), 4.41 (dd, 1H, ³J = 3.15 Hz and ³J = 5.05 Hz, H-3'), 4.18 - 3.99 (AB-system with A d and B d, partially overlapping with 2 x O-CH₂, 2H, ²J = 16.35 Hz, N-CH₂, 2-amidoethanamide), 4.09 - 4.01 (2 x q, 4H, 2 x O-CH₂), 3.29 (s, 3H, 3-CH₃), 3.25 (d, 2H, ²J_{H,P} = 21.45 Hz, CH₂-P, benzylphosphonate), 1.27 (t, 6H, 2 x CH₃).
¹³C-NMR (125 MHz, MeOD), δ 173.2 (C=O), 169.5 (C=O), 165.4 (C-4), 153.1 (C-2), 142.3 (C-6), 138.8 (C-NH, benzylphosphonate), 131.7 (2 x CHₘₑₜₐ, benzylphosphonate), 128.8 (d, ²J_{C,P} = 9.4 Hz, C-CH₂-P, benzylphosphonate), 121.5 (2 x CHₒᵣₜₕₒ, benzylphosphonate), 102.6 (C-5), 93.6 (C-1'), 85.3 (C-4), 74.9 (C-2), 74.4 (C-3), 64.0 (2 x O-CH₂), 43.9 (N-CH₂, 2-amidoethaneamide), 33.4 (d, ¹J_{C,P} = 137.5 Hz, CH₂-P, benzylphosphonate), 28.4 (3-CH₃), 17.0 and 16.9 (2 x CH₃).
³¹P-NMR (202 MHz, MeOD), δ 26.7.

### X. 4-[2-((2S,3R,4S,5R)-5-(2,4-Dioxo-3,4-dihydro-5-methylpyrimidine-1(2H)-yl)-3,4-dihydroxy-tetrahydrofurane-2-carboxamido)ethaneamidolbenzylphosphonic acid diethylester (19)

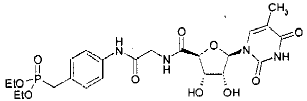

In a dry vessel, N-tert-butyloxycarbonylglycine (10 mmol, 1750 mg) was dissolved in 10 ml of dry THF and cooled to -25 °C. Subsequently, N-methylmorpholine (10 mmol, 1010 mg) and isobutyl chloroformate (10 mmol, 1360 mg) were sequentially added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) is added. The resulting mixture was allowed to warm to rt. After 3 h, the volatiles were removed by rotary evaporation at 40 °C, the residue was dissolved in 10 ml of water and adjusted to pH 1 (10 % aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for 2 h at rt. p-(Aminomethylcarboxamido)benzylphosphonic acid diethyl ester hydrochloride was precipitated by addition of 50 ml of diethyl ether, filtered off and thoroughly washed with diethyl ether (yield over two steps: 3100 mg, 92%, white crystals).

Under an atmosphere of argon, 2',3'-anisylidene-5-methyluridine-4'-carboxylic acid (1 mmol, 390 mg), HCTU^{®} (1.1 mmol, 455 mg) and 1-hydroxybenzotriazole (1.1 mmol, 149 mg) were dissolved in 2 ml of dry DMF at rt. Diisopropylethylamine (1.1 mmol, 143 mg) and, after one minute, p-(aminomethylcarboxamido)benzylphosphonic acid diethyl ester hydrochloride (2 mmol, 672 mg), dissolved in a mixture of dry DMF (2 ml) and diisopropylethylamine (0.5 ml), were added sequentially via a syringe to the solution. Vigorous stirring was continued for 24 h at rt. The volatiles were removed in vacuum at 40 °C and the residue is purified by silica gel column chromatography using dichloromethane : methanol (40 : 1). The product is isolated by rotary evaporation at 40 °C and recrystallized from diethyl ether. Deprotection of the ribose moiety is performed by stirring 2',3'-anisylidene-5-methyluridine-5'-amide (100 mg) in a mixture of dichloromethane (3 ml), trifluoroacetic acid (0.15 ml) and water (one drop) at rt. After 2 h, the crude product was precipitated by the addition of diethyl ether (50 ml), filtered off, dissolved in 7 ml of water : methanol (75 : 25) and purified by RP-HPLC using a gradient of water : methanol from 75 : 25 to water : methanol 0 : 100. 300 mg of the title compound (19) was isolated by lyophilization as white amorphous powder (yield over two steps: 54%).
¹H-NMR (500 MHz, MeOD), δ 7.97 (s, 1H, H-6), 7.58 (d, 2H, ³J = 7.90 Hz, 2 x CHₘₑₜₐ, benzylphosphonate), 7.39 (dd, 2H, ³J = 8.50 Hz and ⁴J = 2.55 Hz, 2 x CHₒᵣₜₕₒ, benzylphosphonate), 6.02 (d, 1H, ³J = 6.95 Hz, H-1'), 4.51 (d, 1H, ³J = 2.55 Hz, H-4'), 4.47 (dd, 1H, ³J = 5.00 Hz and ³J = 6.90 Hz, H-2'), 4.41 (dd, 1H, ³J = 2.50 Hz and ³J = 5.05 Hz, H-3'), 4.24 - 3.99 (AB-system with A d and B d, partially overlapping with 2 x O-CH₂, 2H, ²J = 16.70 Hz, N-CH₂, 2-amidoethanamide), 4.09 - 4.01 (2 x q, 4H, 2 x O-CH₂), 3.25 (d, 2H, ²J_{H,P} = 21.45 Hz, CH₂-P, benzylphosphonate), 1.93 (s, 3H, 5-CH₃), 1.29 (t, 6H, 2 x CH₃).
¹³C-NMR (125 MHz, MeOD), δ 173.2 (C=O), 169.4 (C=O), 166.7 (C-4), 153.3 (C-2), 139.8 (C-NH, benzylphosphonate), 138.9 (C-6), 131.7 (2 x CHₘₑₜₐ, benzylphosphonate), 128.7 (d, ²J_{C,P} = 9.4 Hz, C-CH₂-P, benzylphosphonate), 121.4 (2 x CHₒᵣₜₕₒ, benzylphosphonate), 112.5 (C-5), 91.6 (C-1'), 85.3 (C-4), 75.0 (C-2), 73.7 (C-3), 64.0 (2 x O-CH₂), 43.9 (N-CH₂, 2-amidoethanamide), 33.4 (d, ¹J_{C,P} = 137.5 Hz, CH₂-P, benzylphosphonate), 17.0 and 16.9 (2 x CH₃), 12.6 (5-CH₃).
³¹P-NMR (202 MHz, MeOD), δ 26.7.

### Y. 4-[4-((2S,3R,4S,5R)-5-(2,4-Dioxo-3,4-dihydro-5-methylpyrimidine-1(2H)-yl)-3,4-dihydroxy-tetrahydrofurane-2-carboxamido)butaneamido]benzylphosphonic acid diethylester (20)

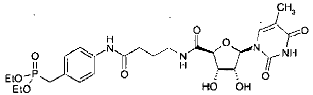

In a dry vessel, N-tert-butyloxycarbonyl-γ-aminobutyric acid (10 mmol, 2030 mg) was dissolved in 10 ml of dry THF and cooled to -25 °C. Subsequently, N-methylmorpholine (10 mmol, 1010 mg) and isobutyl chloroformate (10 mmol, 1360 mg) were sequentially added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) was added. The resulting mixture was allowed to warm to rt. After 3 h, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for 2 h at rt. p-(Aminopropylcarboxamido)benzylphosphonic acid diethyl ester hydrochloride is precipitated by addition of 50 ml of diethyl ether, filtered off and thoroughly washed with diethyl ether (yield over two steps: 3300 mg, 91%, white crystals).

Under an athmosphere of argon, 2',3'-anisylidene-5-methyluridine-5'-carboxylic acid (1 mmol, 390 mg), HBTU^{®} (1.1 mmol, 430 mg) and 1-hydroxybenzotriazole (1.1 mmol, 149 mg) were dissolved in 2 ml of dry DMF at rt. Diisopropylethylamine (1.1 mmol, 143 mg) and, after one minute, p-(aminopropyl-carboxamido)benzylphosphonic acid diethyl ester hydrochloride (2 mmol, 672 mg), dissolved in a mixture of dry DMF (2 ml) and diisopropylethylamine (0.5 ml), were added sequentially via a syringe to the solution. Vigorous stirring was continued for 24 h at rt. The volatiles were removed in vacuum at 40 °C and the residue was purified by silica gel column chromatography using dichloromethane : methanol (40 : 1). The product was isolated by rotary evaporation at 40 °C and recrystallized from diethyl ether. Deprotection of the ribose moiety was performed by stirring 2',3'-anisylidene-5-methyluridine-5'-amide (100 mg) in a mixture of dichloromethane (3 ml), trifluoroacetic acid (0.15 ml) and water (one drop) at rt. After 2 h, the crude product was precipitated by the addition of diethyl ether (50 ml), filtered off, dissolved in 7 ml of water : methanol (75 : 25) and purified by RP-HPLC using a gradient of water : methanol from 75 : 25 to water : methanol 0 : 100. The title compound (20) was isolated by lyophilization.
¹H-NMR (500 MHz, MeOD), δ 7.91 (s, 1H, H-6), 7.58 (d, 2H, ³J = 7.90 Hz, 2 x CHₘₑₜₐ, benzylphosphonate), 7.29 (dd, 2H, ³J = 8.50 Hz and ,⁴J = 2.55 Hz, 2 x CHₒᵣₜₕₒ, benzylphosphonate), 5.85 (d, 1H, ³J = 6.30 Hz, H-1'), 4.47 (dd, 1H, ³J = 5.05 Hz and ³J = 6.00 Hz, H-2'), 4.39 (d, 1H, ³J = 3.15 Hz, H-4'), 4.27 (dd, 1H, ³J = 2.50 Hz and ³J = 5.05 Hz, H-3'), 4.08 - 4.05 (2 x q, 4H, 2 x O-CH₂), 3.35 (t, 2H, ³J = 7.25 Hz, N-CH₂, amidobutanamide), 3.25 (d, 2H, ²J_{H,P} = 21.45 Hz, CH₂-P, benzylphosphonate), 2.47 (t, 2H, ³J = 7.25 Hz, O=C-CH₂, amidobutaneamide), 1.96 (tt, 2H, ³J = 7.25 Hz, CH₂, amidobutaneamide), 1.93 (s, 3H, 5-CH₃), 1.29 (t, 6H, 2 x CH₃).
¹³C-NMR (125 MHz, MeOD), δ 174.2 (C=O), 172.8 (C=O), 166.7 (C-4), 153.1 (C-2), 140.3 (C-NH, benzylphosphonate), 139.1 (C-6), 131.6 (2 x CHₘₑₜₐ, benzylphosphonate), 128.5 (d, ²J_{C,P} = 9.4 Hz, C-CH₂-P, benzylphosphonate), 121.6 (2 x CHₒᵣₜₕₒ, benzylphosphonate), 112.1 (C-5), 92.9 (C-1'), 85.4 (C-4), 74.9 (C-2), 73.8 (C-3), 64.0 (2 x O-CH₂), 40.3 (N-CH₂, amidobutaneamide), 35.5 (O=C-CH₂), 33.4 (d, ¹J_{C,P} = 137.5 Hz, CH₂-P, benzylphosphonate), 26.6 (CH₂, amidobutaneamide), 17.0 and 16.9 (2 x CH₃), 12.6 (5-CH₃).
³¹P-NMR (202 MHz, MeOD), δ 26.9.

### Z. 4-[2-((2S,3R,4S,5R)-5-(2,4-Dioxo-3,4-dihydropyrimidine-1(2H)-yl)-3,4-dihydroxy-tetrahydrofurane-2-carboxamido)ethaneamidolbenzylphosphonic acid (21)

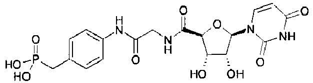

In a dry vessel, N-tert-butyloxycarbonylglycine (10 mmol, 1750 mg) was dissolved in 10 ml of dry THF and cooled to -25 °C. Subsequently, N-methylmorpholine (10 mmol, 1010 mg) and isobutyl chloroformate (10 mmol, 1360 mg) were sequentially added under vigorous stirring. Immediately after the formation of a white precipitate (N-methylmorpholine hydrochloride) a solution of aminobenzylphosphonic acid diethyl ester (11 mmol, 2673 mg) in dry THF (10 ml) was added. The resulting mixture was allowed to warm to rt. After 3 h, the volatiles were removed by rotary evaporation at 40 °C, the residue was dissolved in 10 ml of water and adjusted to pH 1 (10 % aq. NaHSO₄ solution) and extracted with ethyl acetate (3 x 50 ml). The combined organic layers are washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for 2 h at rt. p-(Aminomethylcarboxamido)-benzylphosphonic acid diethyl ester hydrochloride is precipitated by addition of 50 ml of diethyl ether, filtered off and thoroughly washed with diethyl ether(yield over two steps: 3100 mg, 92%, white crystals).

Under an atmosphere of argon, 2',3'-anisylideneuridine-5'-carboxylic acid (1 mmol, 376 mg), HCTU^{®} (1.1 mmol, 455 mg) and 1-hydroxybenzotriazole (1.1 mmol, 149 mg) were dissolved in 2 ml of dry DMF at rt. Diisopropylethylamine (1.1 mmol, 143 mg) and, after one minute, p-(aminomethylcarboxamido)benzylphosphonic acid diethyl ester hydrochloride (2 mmol, 672 mg), dissolved in a mixture of dry DMF (2 ml) and diisopropylethylamine (0.5 ml), were added sequentially via a syringe to the solution. Vigorous stirring was continued for 24 h at rt. The volatiles were removed in vacuum at 40 °C and the residue was purified by silica gel column chromatography using dichloromethane : methanol (40 : 1). The product was isolated by rotary evaporation at 40 °C and recrystallized from diethyl ether. Deprotection of the ribose moiety was performed by stirring 2',3'-anisylideneadenosine-5'-amide (100 mg) in a mixture of dichloromethane (3 ml), trifluoroacetic acid (0.15 ml) and water (one drop) at rt. After 2 h, the crude product was precipitated by the addition of diethyl ether (50 ml), filtered off, dissolved in 7 ml of water : methanol (75 : 25) and purified by RP-HPLC using a gradient of water : methanol from 75:25 to water: methanol 0:100. 4-[2-((2*S*,3*R*,4*S*,S*R*)-5-(2,4-Dioxo-3,4-dihydropyrimidine-1(2*H*)-yl)-3,4-dihydroxy-tetrahydrofurane-2-carboxamido)ethaneamido]benzylphosphonic acid diethylester was isolated by lyophilization (yield over two steps: 310 mg, 68 %, white amorphous powder).

The phosphonic acid ester (16.2 mg, 0.03 mmol) was suspended in 3 ml of dry CH₂Cl₂ at 0°C (ice bath) under argon. Then, trimethylsilyl bromide (0.3 ml, 2.4 mmol) was added dropwise via a syringe. The ensuing clear solution was allowed to warm up slowly to rt and stirred overnight. After 16 h the volatile were removed in vacuum (ice bath) and the residue was dissolved in 2 ml of water, pre-cooled on ice, at 0°C and adjusted to pH 7 using saturated aquaeous NaHCO₃-solution. The product was stirred 2 h in water at 0°C, then adjusted to pH 2 by means of trifluoroacetic acid and purified by RP-HPLC using a gradient of water : methanol from 90 : 10 to water : methanol 0 : 100. The solvents contained 0.1 % of trifluoroacetic acid. Pure title compound (21) was isolated by lyophilisation.
¹H-NMR (500 MHz, D₂O), δ 7.98 (d, 1H, ³J = 7.90 Hz, H-6), 7.39 (d, 2H, ³J = 8.50 Hz, 2 x CHₘₑₜₐ, benzylphosphonate), 7.33 (dd, 2H, ³J = 8.50 Hz and ⁴J = 2.20 Hz, 2 x CHₒᵣₜₕₒ, benzylphosphonate), 5.97 (d, 1H, ³J = 5.05 Hz, H-1'), 5.78 (d, 1H, ³J = 8.20 Hz, H-5), 4.61 (d, 1H, ³J = 3.80 Hz, H-4'), 4.53 (pseudo-t, 1H, ³J = 5.05 Hz and ³J = 5.35 Hz, H-2'), 4.50 (pseudo-t, 1H, ³J = 3.80 Hz and ³J = 5.05 Hz, H-3'), 4.20 - 4.10 (AB-system with A d and B d, 2H, ²J = 16.70 Hz, N-CH₂, amidoethanamide), 3.12 (d, 2H, ²J_{H,P} = 20.80 Hz, CH₂-P, benzylphosphonate).
¹³C-NMR (125 MHz, D₂O), δ 175.0 (C=O), 172.3 (C=O), 169.0 (C-4), 154.6 (C-2), 145.8 (C-6), 137.6 (C_{phenyl}-NH, benzylphosphonate), 134.3 (d, ²J_{C,P} = 9.2 Hz, C_{phenyl}-CH₂-P, benzylphosphonate), 133.1 (2 x CHₒᵣₜₕₒ, benzylphosphonate), 125.0 (2 x CHₘₑₜₐ, benzylphosphonate) 105.4 (C-5), 93.8 (C-1'), 85.6 (C-4), 75.4 (C-2), 75.3 (C-3), 45.5 (N-CH₂, amidoethanamide), 37.4 (d, ¹J_{C,P} = 129.1 Hz, CH₂-P, benzylphosphonate).
³¹P-NMR (202 MHz, D₂O), δ 26.7.
MS (ESI), m/z +1: 541.0, m/z -1: 539.3

### AA. 2-[2-((2S,3R,4S,5R)-5-(2,4-Dioxo-3,4,5,6-tetrahydropyrimidine-1(2H)-yl)-3,4-dihydroxy-tetrahydrofurane-2-carboxamido)ethaneamido]-(S)-aspartic acid (22)

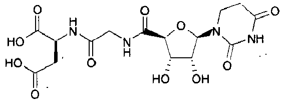

In a dry vessel, N-tert-butyloxycarbonylglycine (10 mmol, 1750 mg) was dissolved in 10 ml of dry THF and cooled to -25 °C. Subsequently, N-methylmorpholine (10 mmol, 1010 mg) and isobutyl chloroformate (10 mmol, 1360 mg) were sequentially added under vigorous stirring. Immediately after the formation of a white precipitate (N-methylmorpholine hydrochloride) a solution of dibenzylaspartate tosylate (11 mmol, 5.3 g), dissolved in 11 ml of 1N NaOH, was added. The resulting mixture is allowed to warm to rt. After 3 h, the volatiles are removed by rotary evaporation at 40 °C, the residue was dissolved in 10 ml of water and adjusted to pH 1 (10 % aq. NaHSO₄ solution) and extracted with ethyl acetate (3 x 50 ml). The combined organic layers are washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for 2 h at rt. (S)-N-(2-aminoacetyl)aspartic acid dibenzylester hydrochloride was precipitated by addition of 50 ml of diethyl ether, filtered off and thoroughly washed with diethyl ether (yield over two steps: 2.8 g, 70%, white crystals).

Under an atmosphere of argon, 2',3'-anisylideneuridine-5'-carboxylic acid (1 mmol, 390 mg), HCTU^{®} (1.1 mmol, 455 mg) and 1-hydroxybenzotriazole (1.1 mmol, 149 mg) were dissolved in 2 ml of dry DMF at rt. Diisopropylethylamine (1.1 mmol, 143 mg) and, after one minute (S)-N-(2-aminoacetyl)aspartic acid dibenzylester hydrochloride (2 mmol, 800 mg), dissolved in a mixture of dry DMF (2 ml) and diisopropylethylamine (0.5 ml), were added sequentially via a syringe to the solution. The solution was stirred at rt overnight. To isolate the product the solvent was removed in vacuum at 40°C, the residue purified by column chromatography (CH₂Cl₂/MeOH 40:1) and the analytically pure product crystallised from ether (yield: 250 mg, 33%).
100 mg of 2',3'-protected uridine-5'-amide were dissolved in 3 ml of dichloromethane, then 0,15 ml of TFA and one drop of water was added. The solution was stirred until completion of the reaction (overnight) at rt. Upon addition of 20 ml of ether the product precipitated, was filtered off and thoroughly washed with ether (yield: 77 mg, 92%).

The dibenzylester of 2-[2-((*2S,3R,4S,5R*)-5-(2,4-Dioxo-5,6-dihydropyrimidine-1(*2H*)-yl)-3,4-dihydroxy-tetrahydrofurane-2-carboxamido)ethaneamido]-(*S*)-aspartic acid (30 mg, 0.061 mmol) was suspended in 2 ml of MeOH and water by sonification (5:1). Then, the catalyst Pd(OH)₂ (5 mg) was added, the vessel thoroughly purged first by argon and then by hydrogen which were applied by means of a hydrogen generator (Hogen GC, Proton Energy Systems, Wallingford, CT, USA). The reaction was performed for 2 h at a pressure of 25 psi at rt. Then, the suspension was filtered and the catalyst thoroughly washed with methanol and water. The washings were added to the filtrate. The solvent was removed by lyophilisation and 20 mg of the title compound (22) was obtained (yield: 92%).
¹H-NMR (500 MHz, D₂O), δ 5.87 (d, 1H, ³J = 6.30 Hz, H-1'); 4.70 (t, 1H, ³J = 6.00 Hz, N-CH, Asp), 4.48 (d, 1H, ³J = 2.20 Hz, H-4'), 4.42 (dd, 1H, ³J = 5.35 Hz and ³J = 6.35 Hz, H-2'), 4.41 (dd, 1H, ³J = 2.20 Hz and ³J = 5.35 Hz, H-3'), 4.05 (AB-system with A d and B d, 2H, ²J = 17.00 Hz, N-CH₂, 2-amidoethaenamide), 3.61 (m, 2H, ³J = 6.60 Hz, N-CH₂, dihydrouracil), 2.93 (d, ³J = 6.00 Hz, O=C-CH₂, Asp), 2.80 (m, 2H, ³J = 6.00 Hz, O=C-CH₂, dihydrouracil).
¹³C-NMR (125 MHz, D₂O), δ 177.9 (C=O), 177.7 (C=O), 176.6 (C=O), 175.1 (C=O), 173.3 (C-4), 157.6 (C-2), 91.8 (C-1'), 84.8 (C-4'), 75.5 (C-2'), 72.5 (C-3'), 49.5 (N-CH, Asp), 45.4 (N-CH₂, 2-amidoethaneamide), 40.7 (N-CH₂, dihydrouracil), 38.9 (O=C-CH₂, Asp), 33.0 (O=C-CH₂, dihydrouracil).

### BB. 2-[4-((2S,3R,4S,5R)-5-(2,4-Dioxo-3,4,5,6-tetrahydropyrimidine-1(2H)-yl)-3,4-dihydroxy-tetrahydrofurane-2-carboxamido)butaneamido]-(S)-aspartic acid (23)

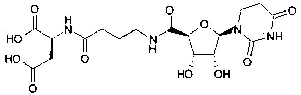

In a dry vessel, N-tert-butyloxycarbonyl-γ-aminobutyric acid (2.1 g, 10 mmol) was dissolved in 10 ml of dry THF and cooled to -25 °C. Subsequently, N-methylmorpholine (10 mmol, 1010 mg) and isobutyl chloroformate (10 mmol, 1360 mg) were sequentially added under vigorous stirring. Immediately after the formation of a white precipitate (N-methylmorpholine hydrochloride) a solution of dibenzylaspartate tosylate (5.3 g, 11 mmol), dissolved in 11 ml of 1N NaOH, was added. The resulting mixture was allowed to warm to rt. After 3 h, the volatiles were removed by rotary evaporation at 40 °C, the residue was dissolved in 10 ml of water and adjusted to pH 1 (10 % aq. NaHSO₄ solution) and extracted with ethyl acetate (3 x 50 ml). The combined organic layers are washed with saturated aq. Na₂CO₃ solution (3 x 20 ml) and subsequently with water (3 x 20 ml), dried over Na₂SO₄, and evaporated to dryness. The residue (boc-protected amide) is dissolved in 8 ml of dry 4N HCl-dioxane solution and stirred for 2 h at rt. (S)-N-(4-aminobutyryl)aspartic acid dibenzylester hydrochloride was precipitated by addition of 50 ml of diethyl ether, filtered off and thoroughly washed with diethyl ether (yield over two steps: 2.3 g (71 %).

Under an atmosphere of argon, 2',3'-anisylideneuridine-5'-carboxylic acid (1 mmol, 390 mg), PyBOP^{®} (1.1 mmol, 572 mg) and 1-hydroxybenzotriazole (1.1 mmol, 149 mg) were dissolved in 2 ml of dry DMF at rt. Diisopropylethylamine (1.1 mmol, 143 mg) and, after one minute(*S*)-N-(4-aminobutyryl)aspartic acid dibenzylester hydrochloride (2 mmol, 866 mg), dissolved in a mixture of dry DMF (2 ml) and diisopropylethylamine (0.5 ml), were added sequentially via a syringe to the solution. The solution was stirred at rt overnight. To isolate the product the solvent was removed in vacuum at 40°C, the residue purified by column chromatography (CH₂Cl₂/MeOH 40:1) and the analytically pure product crystallised from ether (yield: 450 mg, 56%).

100 mg of 2',3'-protected uridine-5'-amide were dissolved in 3 ml of dichloromethane, then 0,15 ml of TFA and one drop of water was added. The solution was stirred until completion of the reaction (overnight) at rt. Upon addition of 20 ml of ether the product precipitated, was filtered off and thoroughly washed with ether (yield: 79 mg, 94 %).

The dibenzylester of 2-[4-((*2S,3R,4S,5R*)-5-(2,4-Dioxo-5,6-dihydropyrimidine-1(*2H*)-yl)-3,4-dihydroxy-tetrahydrofurane-2-carboxamido)butaneamido]-(*S*)-aspartic acid (30 mg, 0.065 mmol) was suspended in 2 ml of MeOH and water by sonification (5:1). Then, the catalyst Pd(OH)₂ (5 mg) was added, the vessel thoroughly purged first by argon and then by hydrogen which were applied by means of a hydrogen generator (Hogen GC, Proton Energy Systems, Wallingford, CT, USA). The reaction was performed for 2 h at a pressure of 25 psi at rt. Then, the suspension was filtered and the catalyst thoroughly washed with methanol and water. The washings were added to the filtrate. The solvent was removed by lyophilisation and 20 mg of the title compound (23) was obtained (yield: 92 %).
¹H-NMR (500 MHz, D₂O) δ 5.82 (d, 1H, ³J = 6.60 Hz, H-1'), 4.71 (t, 1H, ³J = 6.95 Hz, N-CH, Asp), 4.41 (dd, 1H, ³J = 6.60 Hz and ³J = 5.35 Hz, H-2'), 4.38 (d, 1H, ³J = 3.15 Hz, H-4'), 4.35 (dd, 1H, ³J = 3.15 Hz and 5.35 Hz, H-3'), 3.66 (m, 2H, N-CH₂, Dihydrouracil), 3.28 (dt, 2H, ³J = 6.95 Hz and ³J = 7.85 Hz, N-CH₂, 4-amidobutaneamide), 2.91 (AB-system with A dd and B dd, 2H, ³J = 5.05 Hz and ²J = 16.75 Hz, O=C-CH₂, Asp), 2.80 (m, 2H, O=C-CH₂, dihydrouracil), 2.34 (t, 2H, 3J = 7.25 Hz, O=C-CH₂, 4-amidobutaneamide), 1.83 (dt, 2H, ³J = 7.25 Hz and ³J = 6.95 Hz, CH₂, 4-amidobutaneamide).
¹³C-NMR (125 MHz, D₂O) δ 178.4 (C=O), 178.0 (C=O), 177.7 (C=O), 176.6 (C=O), 174.4 (C-4), 157.5 (C-2), 92.0 (C-1'), 84.9 (C-4'), 75.4 (C-2'), 72.5 (C-3'), 52.7 (N-CH, Asp), 41.3 (N-CH₂, dihydrouracil), 40.9 (N-CH₂, 4-amidobutaneamide), 39.1 (O=C-CH₂, dihydrouracil), 35.6 (O=C-CH₂, Asp), 33.0 (O=C-CH₂, 4-amidobutaneamide), 27.5 (CH₂, 4-amidobutaneamide).

### CC. 2-[2-((2S,3R,4S,5R)-5-(2,4-Dioxo-3,4,5,6-dihydropyrimidine-1(2H)-yl)-3,4-dihydroxy-tetrahydrofurane-2-carboxamido)ethaneamido]-(S)-glutamic acid (24)

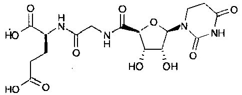

In a dry vessel, N-tert-butyloxycarbonylglycine (2.1 g, 10 mmol) was dissolved in 10 ml of dry THF and cooled to -25 °C. Subsequently, N-methylmorpholine (10 mmol, 1010 mg) and isobutyl chloroformate (10 mmol, 1360 mg) were sequentially added under vigorous stirring. Immediately after the formation of a white precipitate (N-methylmorpholine hydrochloride) a solution of dibenzylglutamic acid hydrochloride (4.0 g, 11 mmol), dissolved in 11 ml of 1N NaOH, was added. The resulting mixture was allowed to warm to rt. After 3 h, the volatiles were removed by rotary evaporation at 40 °C, the residue was dissolved in 10 ml of water and adjusted to pH 1 (10 % aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for 2 h at rt. (S)-N-(4-aminobutyryl)aspartic acid dibenzylester hydrochloride was precipitated by addition of 50 ml of diethyl ether, filtered off and thoroughly washed with diethyl ether (yield over two steps: 3.4 g, 80%).

Under an atmosphere of argon, 2',3'-anisylideneuridine-5'-carboxylic acid (1 mmol, 390 mg), HCTU^{®} (1.1 mmol, 455 mg) and 1-hydroxybenzotriazole (1.1 mmol, 149 mg) are dissolved in 2 ml of dry DMF at rt. Diisopropylethylamine (1.1 mmol, 143 mg) and, after one minute(S)-N-(2-aminoacetyl)glutamic acid dibenzylester hydrochloride (2 mmol, 830 mg), dissolved in a mixture of dry DMF (2 ml) and diisopropylethylamine (0.5 ml), are added sequentially via a syringe to the solution. The solution is stirred at rt overnight. To isolate the product the solvent is removed in vacuum at 40°C, the residue purified by column chromatography (CH₂Cl/MeOH 40:1) and the analytically pure product crystallised from ether (yield: 220 mg, 30 %).

100 mg of 2',3'-protected uridine-5'-amide were dissolved in 3 ml of dichloromethane, then 0,15 ml of TFA and one drop of water was added. The solution was stirred until completion of the reaction (overnight) at rt. Upon addition of 20 ml of ether the product precipitated, was filtered off and thoroughly washed with ether (yield: 79 mg, 94 %).

The dibenzylester of 2-[4-((*2S,3R,4S,5R*)-5-(2,4-Dioxo-5,6-dihydropyrimidine-1(*2H*)-yl)-3,4-dihydroxy-tetrahydrofurane-2-carboxamido)ethaneamido]-(*S*)-glutamic acid (30 mg, 0.062 mmol) was suspended in 2 ml of MeOH and water by sonification (5:1). Then, the catalyst Pd(OH)₂ (5 mg) was added, the vessel thoroughly purged first by argon and then by hydrogen which were applied by means of a hydrogen generator (Hogen GC, Proton Energy Systems, Wallingford, CT, USA). The reaction was performed for 2 h at a pressure of 25 psi at rt. Then, the suspension was filtered and the catalyst thoroughly washed with methanol and water. The washings were added to the filtrate. The solvent was removed by lyophilisation and 20 mg of the title compound (24) was obtained (yield: 92 %).
¹H-NMR (500 MHz, D₂O) δ 5.87 (d, 1H, ³J = 6.00 Hz, H-1'); 4.49 (d, 1H, ³J = 2.50 Hz, H-4'); 4.43 (dd, 1H, ³J = 5.35 Hz und ³J = 6.65 Hz, H-2'), 4.41 (dd, 1H, ³J = 2.50 Hz and ³J = 5.15 Hz, H-3'), 4.35 (t, 1H, ³J = 4.75 Hz, N-CH, Glu); 4.05 (AB-System mit A d und B d, 2H, ²J = 17.00 Hz, N-CH₂, 2-Amidoethanamid); 3.61 (m, 2H, ³J = 6.60 Hz, N-CH₂, Dihydrouracil); 2.80 (dt, 2H, ³J = 6.00 Hz und ³J = 3.75 Hz, O=C-CH₂, Dihydrouracil); 2.56 (t, 2H , ³J = 7.55 Hz, O=C-CH₂, Glu); 2.20 und 1.99 (2 x m, 2 x 1H, ³J = 7.25 Hz und ³J = 4.75 Hz, CH₂, Glu).
¹³C-NMR (125 MHz, D₂O) δ 180.5 (C=O); 179.5 (C=O); 176.6 (C=O); 175.1 (C=O); 173.4 (C-4); 157.6 (C-2); 91.8 (C-1'); 84.8 (C-4'); 75.5 (C-2'); 72.5 (C-3'); 45.4 (N-CH₂, 2-Amidoethanamid); 45.0 (N-CH, Glu); 40.7 (N-CH₂, Dihydrouracil); 33.2 (O=C-CH₂, Dihydrouracil); 33.0 (O=C-CH₂, Glu); 29.3 (CH₂, Glu).

### DD. 2-[4-((2S,3R,4S,5R)-5-(2,4-Dioxo-5-methylpyrimidin-1(2H)-yl)-3,4-dihydroxy-tetrahydrofurane-2-carboxamido)butaneamido]-(S)-aspartic acid (25)

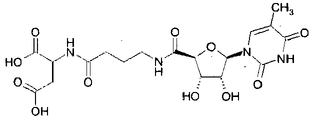

In a dry vessel, N-tert-butyloxycarbonyl-y-aminobutyric acid (2.1 g, 10 mmol) was dissolved in 10 ml of dry THF and cooled to -25 °C. Subsequently, N-methylmorpholine (10 mmol, 1010 mg) and isobutyl chloroformate (10 mmol, 1360 mg) were sequentially added under vigorous stirring. Immediately after the formation of a white precipitate (N-methylmorpholine hydrochloride) a solution of dibenzylaspartate tosylate (5.3 g, 11 mmol), dissolved in 11 ml of 1N NaOH, was added. The resulting mixture was allowed to warm to rt. After 3 h, the volatiles were removed by rotary evaporation at 40 °C, the residue was dissolved in 10 ml of water and adjusted to pH 1 (10 % aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na2SO₄, and evaporated to dryness. The residue (boc-protected amide) is dissolved in 8 ml of dry 4N HCl-dioxane solution and stirred for 2 h at rt. (S)-N-(4-aminobutyryl)aspartic acid dibenzylester hydrochloride was precipitated by addition of 50 ml of diethyl ether, filtered off and thoroughly washed with diethyl ether (yield over two steps: 2.3 g, 71%).

Under an atmosphere of argon, 2',3'-anisylidene-5-methyluridirie-5'-carboxylic acid (1 mmol, 390 mg), PyBOP^{®} (1.1 mmol, 572 mg) and 1-hydroxybenzotriazole (1.1 mmol, 149 mg) were dissolved in 2 ml of dry DMF at rt. Diisopropylethylamine (1.1 mmol, 143 mg) and, after one minute(S)-N-(4-aminobutyryl)aspartic acid dibenzylester hydrochloride (2 mmol, 866 mg), dissolved in a mixture of dry DMF (2 ml) and diisopropylethylamine (0.5 ml), were added sequentially via a syringe to the solution. The solution was stirred at rt overnight. To isolate the product the solvent was removed in vacuum at 40°C, the residue purified by column chromatography (CH₂Cl₂/MeOH 40:1) and the analytically pure product crystallised from ether (yield: 450 mg, 58 %).

100 mg of 2',3'-protected 5-methyluridine-5'-amide were dissolved in 3 ml of dichloromethane, then 0,15 ml of TFA and one drop of water was added. The solution was stirred until completion of the reaction (overnight) at rt. Upon addition of 20 ml of ether the product precipitated, was filtered off and thoroughly washed with ether (yield: 80 mg, 92%).

The dibenzylester of 2-[4-((*2S,3R,4S,5R*)-5-(2,4-Dioxo-5-methylpyrimidine-1(*2H*)-yl)-3,4-dihydroxytetrahydrofurane-2-carboxamido)butaneamido]-(*S*)-aspartic acid (30 mg, 0.062 mmol) was suspended in 2 ml of MeOH and water by sonification (5:1). Then, the catalyst Pd(OH)₂ (5 mg) was added, the vessel thoroughly purged first by argon and then by hydrogen which were applied by means of a hydrogen generator (Hogen GC, Proton Energy Systems, Wallingford, CT, USA). The reaction was performed for 2 h at a pressure of 25 psi at rt. Then, the suspension was filtered and the catalyst thoroughly washed with methanol and water. The washings were added to the filtrate. The solvent was removed by lyophilisation and 21 mg of the title compound (25) was obtaind (yield: 95 %).
¹H-NMR (500 MHz, D₂O) δ 7.79 (s, 1H, H-6), 5.89 (d, 1H, ³J = 6.60 Hz, H-1'), 4.71 (t, 1H, ³J = 6.95 Hz, N-CH, Asp), 4.52 (dd, 1H, ³J = 5.35 Hz and ³J = 6.60 Hz, H-2'), 4.47 (d, 1H, ³J = 2.20 Hz, H-4'), 4.43 (dd, 1H, ³J = 3.80 Hz and ³J = 3.15 Hz, H-3'), 3.28 (dt, 2H, ³J = 6.95 Hz and ³J = 7.85 Hz, N-CH₂, 4-butanamide), 2.91 (AB-system with A dd and B dd, 2H, ³J = 5.05 Hz and ²J = 16.75 Hz, O=C-CH₂, Asp), 2.34 (t, 2H, ³J = 7.25 Hz, O=C-CH₂, 4-butanamide), 1.91 (s, 3H, 5-CH3), 1.85 (dt, 2H, ³J = 7.25 Hz and ³J = 6.95 Hz, CH₂, 4-butanamide). ¹³ C-NMR (125 MHz, D₂O) δ 178.5 (C=O), 177.5 (C=O), 177.5 (C=O), 174.3 (C=O), 169.4 (C-4), 154.7 (C-2), 141.6 (C-6), 114.4 (C-5), 92.0 (C-1'), 84.9 (C-4'), 75.4 (C-2'), 72.5 (C-3'), 52.7 (N-CH, Asp), 40.9 (N-CH₂, 4-butanamide), 35.6 (O=C-CH₂, Asp), 33.0 (O=C-CH₂, 4-butanamide), 27.5 (CH₂, 4-butanamide).

### EE. 2-[4-((2S,3R,4S,5R)-5-(6-Oxo-9H-purine-9-yl)-3,4-dihydroxy-tetrahydrofurane-2-carboxamido)butaneamido]-(S)-aspartic acid (26)

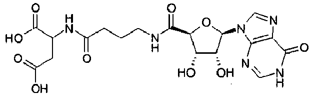

In a dry vessel N-tert-butyloxycarbonyl-γ-aminobutyric acid (2.1 g, 10 mmol) was dissolved in 10 ml of dry THF and cooled to -25 °C. Subsequently, N-methylmorpholine (10 mmol, 1010 mg) and isobutyl chloroformate (10 mmol, 1360 mg) were sequentially added under vigorous stirring. Immediately after the formation of a white precipitate (N-methylmorpholine hydrochloride) a solution of dibenzylaspartate tosylate (5.3 g, 11 mmol), dissolved in 11 ml of 1N NaOH, was added. The resulting mixture was allowed to warm to rt. After 3 h, the volatiles were removed by rotary evaporation at 40 °C, the residue was dissolved in 10 ml of water and adjusted to pH 1 (10 % aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for 2 h at rt. (S)-N-(4-aminobutyryl)aspartic acid dibenzylester hydrochloride was precipitated by addition of 50 ml of diethyl ether, filtered off and thoroughly washed with diethyl ether (yield over two steps: 2.3 g, 71%).

Under an atmosphere of argon, 2',3'-anisylidene-inosine-5'-carboxylic acid (1 mmol, 399 mg), PγBOP^{®} (1.1 mmol, 572 mg) and 1-hydroxybenzotriazole (1.1 mmol, 149 mg) were dissolved in 2 ml of dry DMF at rt. Diisopropylethylamine (1.1 mmol, 143 mg) and, after one minute(*S*)-N-(4-aminobutyryl)aspartic acid dibenzylester hydrochloride (2 mmol, 866 mg), dissolved in a mixture of dry DMF (2 ml) and diisopropylethylamine (0.5 ml), were added sequentially via a syringe to the solution. The solution is stirred at rt overnight. To isolate the product, the solvent was removed in vacuum at 40°C, the residue purified by column chromatography (CH₂Cl₂/MeOH 40:1) and the analytically pure product crystallised from ether (yield: 280 mg, 36 %).

100 mg of 2',3'-protected adenosine-5'-amide were dissolved in 3 ml of dichloromethane, then 0,15 ml of TFA and one drop of water was added. The solution was stirred until completion of the reaction (overnight) at rt. Upon addition of 20 ml of ether the product precipitated, was filtered off and thoroughly washed with ether (yield: 75 mg, 87 %).

The dibenzylester (30 mg, 0.06 mmol) was suspended in 2 ml of MeOH and water by sonification (5:1). Then, the catalyst Pd(OH)₂ (5 mg) was added, the vessel thoroughly purged first by argon and then by hydrogen which were applied by means of a hydrogen generator (Hogen GC, Proton Energy Systems, Wallingford, CT, USA). The reaction was performed for 2 h at a pressure of 25 psi at rt. Then, the suspension was filtered and the catalyst thoroughly washed with methanol and water. The washings were added to the filtrate. The solvent was removed by lyophilisation and 21 mg of the title compound (26) was obtained (yield: 95 %).
¹H-NMR (500 MHz, D₂O) δ 8.57 (H-2), 8.46 (H-8), 6.22 (d, 1H, ³J = 6.60 Hz, H-1'), 4.89 (t, 1H, ³J = 6.95 Hz, N-CH, Asp), 4.72·(dd, 1H, ³J = 5.35 Hz and ³J = 6.60 Hz, H-2'), 4.64 (d, 1H, ³J = 2.20 Hz, H-4'), 4.63 (dd, 1H, ³J = 3.80 Hz and ³J = 3.15 Hz, H-3'), 3.28 (dt, 2H, ³J = 6.95 Hz and ³J = 7.85 Hz, N-CH₂, 4-butanamide), 2.92 (AB-system with A dd and B dd, 2H, ³J = 5.05 Hz and ²J = 16.75 Hz, O=C-CH₂, Asp), 2.32 (t, 2H, ³J = 7.25 Hz, O=C-CH₂, 4-butanamide), 1.81 (dt, 2H, ³J = 7.25 Hz and ³J = 6.95 Hz, CH₂, 4-butanamide).
¹³C_NMR (125 MHz, D₂O) δ 178.5 (C=O), 177.3 (C=O), 177.2 (C=O), 174.0 (C=O), 153.2 (C-6), 151.2 (C-2), 147.8 (C-4), 146.5 (C-8), 122.2 (C-5), 9170 (C-1'), 86.7 (C-4'), 75.8 (C-2'), 75.7 (C-3'), 52.7 (N-CH, Asp), 40.9 (N-CH₂, 4-butanamide), 35.6 (O=C-CH₂, Asp), 33.0 (O=C-CH₂, 4-butanamide), 27.5 (CH₂, 4-butanamide).

### FF. 4-[(25,3R,4S,5R)-5-(2,4-Dioxo-3,4-dihydro-5-methylpyrimidin-1(2H)-yl)-3,4-dihydroxy-tetrahydrofurane-2-carboxamido]butaneamidomethylenediphosphonic acid tetraethylester (27)

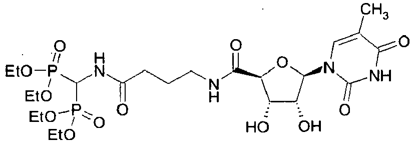

In a dry vessel, N-tert-butyloxycarbonyl-γ-aminobutyric acid (10 mmol, 2030 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of of aminomethylenediphosphonic acid diethylester (11 mmol, 3350 mg) in dry THF (10 ml) was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. 3-Aminopropylcarboxamidomethyl-bis(phosphonic acid diethyl ester) hydrochloride was precipitated by addition of 50 ml of diethyl ether, filtered off and thoroughly washed with diethyl ether (yield over two steps: 3400 mg, 80 %, clay).

Under an atmosphere of argon, 2',3'-anisylidene-5-methyluridine-4'-carboxylic acid (1 mmol, 390 mg), PyBOP^{®} (1.1 mmol, 572 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, 3-aminopropylcarboxamidomethyl-bis(phosphonic acid diethyl ester) hydrochloride (2 mmol, 844 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuo at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylidene-5-methyluridine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 250 mg of the title compound was isolated by lyophilisation as white amorphous powder (yield over two steps: 39 %).
¹H-NMR (500 MHz, MeOD) δ 7.82 (s, 1H, H-6), 5.70 (d, 1H, ³J = 6.95 Hz, H-1'), 5.13 (t, 1H, ²J_{H},_{P} = 22.40 Hz, methylenediphosphonate), 4.66 (dd, 1H, ³J = 6.60 Hz and ³J = 5.05 Hz, H-2'), 4.39 (d, 1H, ³J = 2.50 Hz, H-4'), 4.26-4.21 (m, 9H, H-3' and 4 x O-CH₂), 3.48-3.43 and 3.25-3.11 (2 x m, 2H, N-CH₂, butaneamide), 2.45-2.35 (m, 2H, O=C-CH₂, butaneamide), 1.95 (s, 3H, 5-CH₃), 1.90 (m, 2H, CH₂, butaneamide), 1.40-1.34 (m, 12H, 4 x CH₃).
¹³C-NMR (125 MHz, MeOD) δ 175.1 (C=O), 172.7 (C=O), 166.4 (C-4), 153.4 (C-2), 141.6 (C-6), 112.3 (C-5), 94.8 (C-1'), 85.8 (C-4'), 75.0 (C-2'), 72.7 (C-3'), 65.4 (4 x O-CH₂), 44.8 (t, ¹J_{C,P} = 148.9 Hz, PPNCH, methylenediphosphonate), 39.4 (N-CH₂, butaneamide), 34.0 (O=C-CH₂, butaneamide), 27.2 (CH₂, butaneamide), 16.9 (4 x CH₃), 12.6 (5-CH₃).
³¹P-NMR (202 MHz, MeOD) 15.8.

### GG. (R,S)-4-[(2S,3R,4S,5R)-5-(2,4-Dioxo-3,4-dihydropyrimidin-1(2H)-yl)-3,4-dihydroxy-tetrahydrofurane-2-carboxamido]butaneamido-phenyl-methylphosphonic acid diethylester (28)

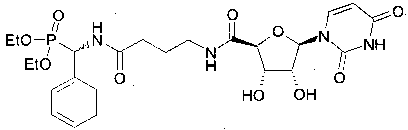

In a dry vessel, N-tert-butyloxycarbonyl-γ-aminobutyric acid (10 mmol, 2030 mg) was dissolved in 10 ml of dry THF and cooled to -25 °C. Subsequently, N-methylmorpholine (10 mmol, 1010 mg) and isobutyl chloroformiate (10 mmol, 1360 mg) were sequentially added under vigorous stirring. Immediately after the formation of a white precipitate (N-methylmorpholine hydrochloride) a solution of (R,S)-a-amino-benzylphosphonic acid diethyl ester hydrochloride (11 mmol, 3080 mg) in THF (10 ml) and 1N aq. NaOH (11 ml), pre-cooled on ice, was added. The resulting mixture was allowed to warm to ambient temperature. After three hours, the volatiles were removed by rotary evaporation at 40 °C, the residue was dissolved in 10 ml of water and adjusted to pH 1 (10% aq. NaHSO₄ solution) and extracted with ethyl acetate (3 x 50 ml). The combined organic layers were washed with saturated aq. Na₂CO₃ solution (3 x 20 ml) and subsequently with water (3 x 20 ml), dried over Na₂SO₄, and evaporated to dryness. The residue (boc-protected amide) was dissolved in 8 ml of dry 4N HCl-dioxane solution and stirred for two hours at ambient temperature. (R,S)-α-(3-Aminopropylcarboxamido)benzylphosphonic acid diethyl ester hydrochloride was precipitated by addition of 50 ml of diethyl ether, filtered off and thoroughly washed with diethyl ether (yield over two steps: 3120 mg, 86 %, white crystals).

Under an atmosphere of argon, 2',3'-anisylideneuridine-4'-carboxylic acid (1 mmol, 376 mg), PyBOP^{®} (1.1 mmol, 455 mg) and 1-hydroxybenzotriazole (1.1 mmol, 149 mg) were dissolved in 2 ml of dry DMF at ambient temperature. Diisopropylethylamine (1.1 mmol, 143 mg) and, after one minute, (R,S)-α-(3-Aminopropylcarboxamido)benzylphosphonic acid diethyl ester hydrochloride (2 mmol, 726 mg), dissolved in a mixture of dry DMF (2 ml) and diisopropylethylamine (0.5 ml), were added sequentially via a syringe to the solution. Vigorous stirring was continued for 24 hours at ambient temperature. The volatiles were removed in vacuo at 40 °C and the residue was purified by silica gel column chromatography using dichloromethane/methanol (40:1). The product was isolated by rotary evaporation at 40 °C and recrystallized from diethyl ether. Deprotection of the ribose moiety was performed by stirring 2',3'-anisylideneuridine-5'-amide (100 mg) in a mixture of dichloromethane (3 ml), trifluoroacetic acid (0.15 ml) and water (0.1 ml) at ambient temperature. After two hours, the crude product was precipitated by addition of diethyl ether (50 ml), filtered off, dissolved in 7 ml of water/methanol (75:25) and purified by RP-HPLC using a gradient of water/methanol from 75:25 to water/methanol 0:100. 310 mg of the title compound as a racemic mixture was isolated by lyophilisation as white amorphous powder (yield over two steps: 63%).
¹H-NMR (500 MHz, MeOD) δ 8.11 (2 x d, 1H, ³J = 7.85 Hz, H-6), 7.52 (d, 2H, ³J = 7.85 Hz, 2 x Hₒᵣₜₕₒ, phenyl), 7.42-7.34 (m, 3H, 2 x Hₘₑₜₐ and Hₚₐᵣₐ, phenyl), 5.83 (2 x d, 1H, ³J = 6.00 Hz, H-1'), 5.78 (2 x d, 1H, ³J = 8.20 Hz, H-5), 5.59 (d, 1H, ²J_{H,P} = 21.40 Hz, H_{α}, α-aminobenzylphosphonate), 4.52 (pseudo-q, 1H, ³J = 5.05 Hz and ³J = 6.65 Hz, H-2'), 4.39 (2 x d, 1H, ³J = 3.15 Hz, H-4'), 4.27 (2 x dd, 1H, ³J = 5.05 Hz and ³J = 3.15 Hz, H-3'), 4.16-4.11 (dq, 2H, O-CH₂), 4.02 (m, 1H, 0.5 x O-CH₂), 3.92 (m, 1H, 0.5 x O-CH₂), 3.38-3.26 (m, partly below solvent peak, 2H, N-CH₂, butaneamide), 2.34 (m, 2H, O=C-CH₂, butaneamide), 1.88 (m, 2H, CH₂, butaneamide), 1.36 (2 x t, 6H, 2 x CH₃).
¹³C-NMR (125 MHz, MeOD) δ 175.1 and 175.0 (C=O), 172.7 (C=O), 166.3 and 166.2 (C-4), 153.1 and 153.0 (C-2), 145.0 and 144.9 (C-6), 136.4 (Cᵢₚₛₒ, phenyl), 129.9-129.6 (5 x C, phenyl), 103.4 and 103.3 (C-5), 93.7 and 93.6 (C-1'), 85.5and 85.4 (C-4'), 74.9 and 74.8 (C-2'), 73.9 and 73.8 (C-3'), 65.0 and 64.9 (2 x O-CH₂), 52.7 (2 x d, ¹J_{c,p}= 156.8 Hz, CH-P, benzylphosphonate), 39.9 and 39.8 (N-CH₂, butaneamide), 34.3 and 34.2 (O=C-CH₂, butaneamide), 27.1 and 27.0 (CH₂, butaneamide), 17.1 and 16.8 (2 x CH₃).
³¹P-NMR (202 MHz, MeOD) δ 20.8.

### Example 2: NTPDase assays by capillary electrophoresis (CE)

The applied enzyme inhibition assay has been described (Iqbal J, Vollmayer P, Braun N, Zimmermann H, Müller CE. A capillary electrophoresis method for the characterization of ecto-nucleoside triphosphate diphosphohydrolases (NTPDases) and the analysis of inhibitors by in-capillary enzymatic microreaction. Purinergic Signalling 2005, 1, 349-358).
A. CE instrumentation: All experiments were carried out using a P/ACE MDQ capillary electrophoresis system (Beckman Instruments, Fullerton, CA, USA) equipped with a UV detection system coupled with a diode-array detector (DAD). Data collection and peak area analysis were performed by the P/ACE MDQ software 32 KARAT obtained from Beckman Coulter. The capillary temperature was kept constant at 25 °C. The temperature of the sample storing unit was also adjusted to 25 °C. The electrophoretic separations were carried out using an eCAP polyacrylamide-coated fused-silica capillary [(30 cm (20 cm effective length) x 50 µm internal diameter (I.D.) x 360 µm outside diameter (O.D.), obtained from CS-Chromatographie (Langerwehe, Germany)]. The separation was performed using an applied current of -60 µA and a data acquisition rate of 8 Hz. Analytes were detected using direct UV absorbance at 210 nm. The capillary was conditioned by rinsing with water for 2 min and subsequently with buffer (phosphate 50 mM, pH 6.5) for 1 min. Sample injections were made at the cathodic side of the capillary.
B. NTPDase inhibition assay by capillary electrophoresis: Enzyme inhibition assays were carried out at 37 °C in a final volume of 100 µl. The reaction mixture contained 320 µM of ATP (substrate) in reaction buffer. The reaction buffer contained 140 mM NaCl, 5 mM KCl, 1 mM MgCl₂, 2 mM CaCl₂, and 10 mM Hepes, pH 7.4.

Different concentrations of inhibitors dissolved in DMSO or water (10 µl) were added and the reaction was initiated by the addition of 10 µl of the appropriately diluted recombinant or native human or rat NTPDase enzyme. The final DMSO concentration did not exceed 1 %. The mixture (final volume 100 µl) was incubated for 15 min and terminated by heating at 99 °C for 4 min. Aliquots of the reaction mixture (50 µl) were then transferred to mini-CE vials and injected into the CE instrument under the conditions described above. 20 µM of UMP was used as internal standard. Inhibition of NTPDase was tested over a range of 6 to 8 concentrations of test compound spanning 3 orders of magnitude to determine Kᵢ values. Each analysis was repeated two times (in triplicates) in two separate experiments. The Cheng-Prusoff equation was used to calculate the Kᵢ values from the IC₅₀ values, determined by the non-linear curve fitting program PRISM^{®} 3.0 (GraphPad, San Diego, California, USA). In table 1 Kᵢ values of selected compounds as inhibitors of human NTPDases are given.

**Table 1: Kᵢ values for human NTPDase inhibition obtained for selected new inhibitors using the capillary electrophorese (CE) method. The results were means ± SEM of three separate experiments each run in triplicate¹.**

| Compound No. | NTPDase1 Kᵢ [µM] ± SEM | NTPDase2 Kᵢ [µM] ± SEM | NTPDase3 Kᵢ [µM] ± SEM | NTPDase8 Kᵢ [µM] ± SEM |
|---|---|---|---|---|
| 1 | n.d.² | 117 ±15 | n.d.² | n.d.² |
| 5 | 786 ± 33 | 71.7 ± 13.50 | >> 200 (0)³ | >> 100 (0)³ |
| 2 | >> 50 (50)³ | 8.2 ± 2.1 | >> 200 (0)³ | >> 100 (0)³ |
| 8 | >> 50 (0)³ | 116 ± 24 | >> 200 (22)³ | >> 100 (0)³ |
| 6 | >> 50 (0)³ | 167 ± 21 | >> 200 (0)³ | >> 100 (0)³ |
| 4 | 182 ± 24.3 | 210 ± 25.3 | >> 200 (48)³ | 242 ± 39.3 |
| 3 | 55.2 ± 2.6 | 173 ± 17 | >> 200 (40)³ | >> 100 (0)3 |
| 7 | >> 50 (45)³ | 29.2 ± 2.7 | >> 200 (0)³ | >> 100 (0)3 |
| 9 | n.d.² | ca. 165 (54)³ | n.d.² | n.d.² |
| 12 | 32.4 ± 1.1 | ca. 165 (54)³ | n.d.² | n.d.² |
| 11 | 325 ± 25 | ca. 175 (51)³ | n.d.² | n.d.² |
| 10 | 93 ± 14 | ca. 165 (54)³ | n.d.² | n.d.² |
| CC1 | >> 50 (0)³ | >> 200 (0)³ | >> 200 (0)³ | >> 100 (0)³ |
| CC3 | 161 ± 24 | 213 ± 34 | >> 200 (0)³ | >> 100 (0)³ |
| CC2 | 154 ± 37 | 1440 ± 64 | » 200 (0)³ | 255 ± 25.3 |
| CC4 | >> 50 (0)³ | >> 200 (0)³ | n.d.² | n.d.² |
| CC5 | >> 50 (0)³ | >> 200 (0)³ | n.d.² | n.d.² |
| 15 | 29 ± 4 | n.d.² | n.d.² | n.d.² |
| 13 | 55 ± 5 | n.d.² | n.d.² | n.d.² |
| 14 | 10.8 ± 1 | n.d.² | n.d.² | n.d.² |

| | | | | |
|---|---|---|---|---|
| ¹⁾ Kₘ (NTPDase1): 17 µM; Kₘ (NTPDase2): 70 µM; Kₘ (NTPDase3): 75 µM; Kₘ (NTPDase8): 46 µM; concentration of ATP: 320 µM. ²⁾ not determined ³⁾ inhibition (%) at 1 mM | | | | |

In table 2 inhibition data of selected examples at human P2Y receptor subtypes were collected. It can be seen that no compound was identified that inhibited P2Y receptors at high concentrations of 100 µM and 10 µM, respectively.

**Table 2: Percent inhibition values at selected P2Y receptor subtypes. The results were means ± SEM of three separate experiments each run in triplicate.**

| Compound No. | hP2Y₂ % inhibition of UTP binding at 100 µM, n = 3, Ø | hP2Y₄ % inhibition of UTP binding at 100 µM; n = 3, Ø | rP2Y₆ % inhibition of UDP binding at 100 µM, n = 3, Ø | hP2Y₁₂ % Inhibition of [³H]PSB-0413 Binding at 10µM, n=3, Ø |
|---|---|---|---|---|
| 1 | n.d. | n.d. | - 12 ± 3 | n.d. |
| 5 | n.d. | n.d. | 17 ± 16 | 31 ± 1 |
| 2 | n.d. | n.d. | -10 ± 10 | 2 ± 6 |
| 8 | n.d. | n.d. | 9 ± 13 | 2 ± 3 |
| 6 | n.d. | n.d. | - 3 ± 2 | 7 ± 5 |
| 4 | n.d. | n.d. | - 8 ± 11 | 6 ± 4 |
| 3 | n.d. | 14 ± 6 | 7 ± 7 | 9 ± 4 |
| 7 | n.d. | n.d. | - 3 ± 11 | 2 ± 8 |
| 9 | n.d. | n.d. | n.d. | n.d. |
| 12 | - 44 ± 20 | 27 ± 7 | 18 ± 4 | 1 ± 5 |
| 11 | - 12 ± 24 | 7 ± 20 | 14 ± 2 | 1 ± 5 |
| 10 | - 38 ± 11 | 34 ± 15 | 8 ± 14 | 2 ± 5 |
| CC1 | n.d. | n.d. | n.d. | -4 ± 6 |
| CC3 | n.d. | n.d. | n.d. | 5 ± 1 |
| CC2 | n.d. | n.d. | n.d. | 1 ± 6 |
| CC4 | n.d. | n.d. | 5 ± 8 | n.d. |
| CC5 | n.d. | n.d. | -5 ± 2 | n.d. |
| 15 | -28 ± 11 | n.d. | n.d. | n.d. |
| 13 | n.d. | n.d. | n.d. | 2 ± 5% |
| 14 | n.d. | n.d. | n.d. | 6 ± 4 % |

| | | | | |
|---|---|---|---|---|
| n.d. = not determined | | | | |

**Table 3: Kᵢ values at rat NTPDase1, 2 and 3 and at selected P2Y receptor subtypes obtained for standard compounds: reactive blue 2 (RB2), PPADS, suramin, and ARL67156, using the in-capillary electrophoresis method. The results were means ± SEM of three separate experiments each run in duplicate.**

| Inhibitor | *Kᵢ* ± *SEM [*µ*M]* | | | | | | |
|---|---|---|---|---|---|---|---|
| | *Ectonucleotidases* | | | *selected P2Y-Receptors* | | | |
| | NTPDases1 | NTPDase2 | NTPDase3 | P2Y₂ | P2Y₄ | P2Y₆ | P2Y₁₂ |
| RB2 | 20.0 ± 0.003 | 24.2 ± 0.06 | 1.10 ± 0.03 | 1 | > 100 | 31 | 1.3 |
| PPADS | 46.0 ± 0.01 | 44.2 ± 0.03 | 3.0 ± 0.001 | inactive | 73 % inhib. at 100 µM | 69 % inhib. at 100 µM | |
| Suramin | 300 ± 0.1 | 65.4 ± 0.01 | 12.7 ± 0.03 | 50 | inactive | > 100 | 4.0 |
| ARL 67156 | 27.0 ± 0.004 | >> *600* | 112.1 ± 0.05 | | | | |

**Table 4: Potencies of standard E-NTPDase inhibitors at selected P2X receptor subtypes**

| Inhibitor | Kᵢ ± SEM [µM] | | | |
|---|---|---|---|---|
| | selected P2Y-Receptors | | | |
| | P2X₁ | P2X₂ | P2X₃ | P2X₅ |
| RB2 | 2¹⁵ | 0.4¹⁵ | 50¹⁵ | 20¹⁵ |
| PPADS | 0.13¹⁵ | 1.6¹⁵ | 0.2¹⁵ | 0.2¹⁵ |
| Suramin | 2¹⁵ | 10¹⁵ | 4¹⁵ | 1.6¹⁵ |
| ARL 67156 | | | | |

In tables 3 and 4 data for standard NTPDase inhibitors were collected. For structures of the compounds see fig. 1. It can be seen that - with the exception of the ATP analog ARL 67156 the compounds were non-selective inhibitors of NTPDase1,2 and 3, and they were at least equally potent as antagonists at one or several P2 receptor subtypes. ARL 6,7156 has the disadvantage of being metabolically unstable towards ecto-nucleotide pyrophosphatases (E-NPP). It can be applied as a pharmacological tool but was not suitable in assays where the luciferase assay was used for the quantification of ATP concentrations since it interferes with that assay. One of the new compounds (AMB246.1, Example 2) presented in this patent has been shown not to interfere with the luciferase assay for ATP determination and to have therefore decisive advantages as pharmacological tool.

### Example 3: Ecto-5'-nucleotidase assay

Catalytically active recombinant soluble glutathione-S-transferase/ecto-5'-nucleotidase fusion protein was expressed in insect cells using the baculovirus system and purified by affinity chromatography using agarose-coupled GSH as previously described [Servos, J., Reilander, H., Zimmermann, H. Drug. Dev. Res. 1998, 45, 269-276]

Enzyme assays were carried out at 37 °C in a final volume of 100 µl. The reaction buffer consisted of 10 mM Hepes (2.38 g/L), 2 mM MgCl₂ (0.41 g/L), and 1 mM CaCl₂ (0.11 g/L), brought to pH 7.4 by adding the appropriate amount of 1-N aqueous HCl solution. The reaction was initiated by the addition of 10 µl of the appropriately diluted enzyme (0.52 µg). The reaction mixture was incubated for 10 min and terminated by heating at 99 °C for 5 min. Nucleosides and nucleotides were stable under these conditions. Aliquots of the reaction mixture (50 µl) were then transferred to mini-CE vials containing 50 µl of the internal standard uridine (final concentration 20 µM). In the absence of an internal standard similar results were obtained. Each analysis was repeated twice (duplicates) in three separate experiments.

CE separations were carried out using a P/ACE MDQ system (Beckman Coulter Instruments, Fullerton, CA, USA) equipped with a DAD detection system. The electrophoretic separations were carried out using an eCAP fused-silica capillary [30 cm (20 cm effective length) x 75 µm internal diameter (I.D), x 375 µm outside diameter (O.D) obtained from Beckman Coulter]. The following conditions were applied: T = 25 °C, λₘₐₓ = 260 nm, voltage = 15 kV, running buffer 40 mM sodium borate buffer, pH 9.1. The capillary was washed with 0.1 M NaOH for 2 min, deionized water for 1 min, and running buffer for 1 min before each injection. Injections were made by applying 0.1 psi of pressure to the sample solution for 30 s. The amount of adenosine formed was determined. The CE instrument was fully controlled through a personal computer, which operated with the analysis software 32 KARAT obtained from Beckman Coulter. Electropherograms were evaluated using the same software.

**Table 5: Kᵢ values for rat ecto-5'-nucleotidase inhibition obtained for selected compounds using a capillary electrophoresis (CE) method. The results are means ± SEM of three separate experiments each run in triplicate.**

| Example No. | Rat Ecto-5'-NT Kᵢ [µM] ± SEM |
|---|---|
| 25 | 1.61 ± 0.62 |
| 13 | 0.60 ± 0.01 |
| 23 | 0.180 ± 0.014 |
| 24 | 34.3 ± 0.2 |
| 14 | 0.78 ± 0.18 |
| 21 | 6.47 ± 1.31 |

## Claims

1. A compound represented by the formula (I) 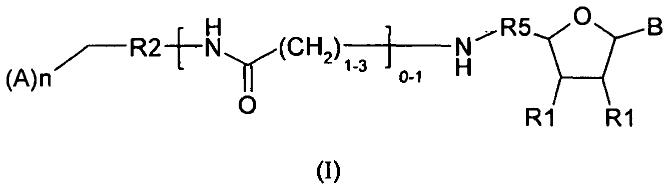 wherein
B represents an oxopurinyl or oxopyrimidinyl residue which is connected with the furanoside ring via one of its nitrogen atoms;
R1 represent independently from each other residues selected from hydroxyl, hydrogen, C₁-C₃-alkoxyl, C₁-C₃-alkyl, C₁-C₃-alkenyl, C₁-C₃-alkinyl, C₁-C₃-acyl, halogen, or commonly form a double bond with one of the vicinal C atoms or an acetyl or ketal ring with each other;
R2 is -(CH₂)₀₋₂- or phenylene;
A represents a -PO(OR3)₂ or -SO2(OR3) residue wherein R3 is C₁-C₃-alkyl, aryl, arylalkyl or heteroary,l or A represents a -(CH₂)ₘ-COOR4 residue, wherein m is an integer from 0 to 2 and R4 is selected from hydrogen and C₁-C₃-alkyl;
R5 is a carbonyl or a methylene group; and
n is 1 or 2, provided that when A represents a -(CH₂)ₘ-COOR4 residue, n is 2;
or a salt thereof.

2. The compound of claim 1, which is represented by the formula 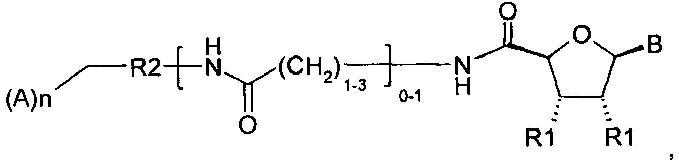 wherein B, R1, R2, n and A are as defined in claim 1.

3. The compound of claim 1 or 2, wherein
(i) at least one R1 is OH and the other R1 is H or OH; and/or
(ii) the spacer between the nucleosid 5'C and A comprises at least three carbon or heteroatoms.

4. The compound of any one of claims 1 to 3, wherein
B is selected from a native oxopurinyl or oxopyrimidyl residue including uracilyl, cytosinyl, guaninyl, hypoxanthinyl, xanthinyl,
preferably B is uracilyl or, most preferably is 1-uracilyl.

5. The compound of any one of claims 1 to 4, wherein A represents a -PO(OR3)₂ residue, R3 is ethyl and/or n is 1.

6. The compound of claim 5, which is represented by the formula 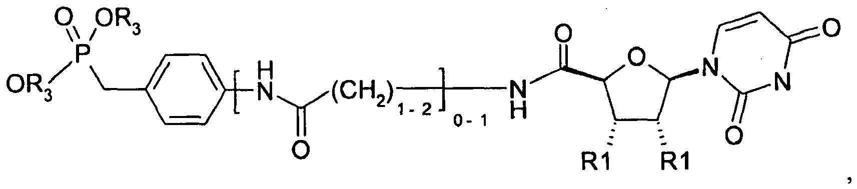 and wherein preferably
(i) at least one R1 is OH and the other R1 is H or OH, more preferably both R1 are OH; and/or
(ii) R3 is ethyl.

7. The compound of claim 6, which is represented by the formula

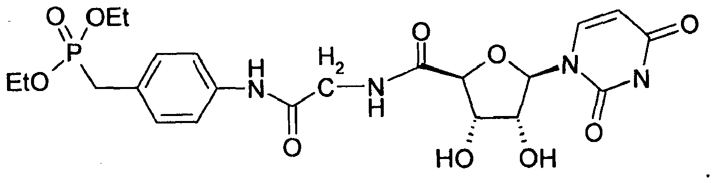

8. The compound of any one of claims 1 to 4, wherein A represents a -(CH₂)ₘ-COOR4 residue and R4 is H.

9. The compound of claim 8, which is represented by the formula

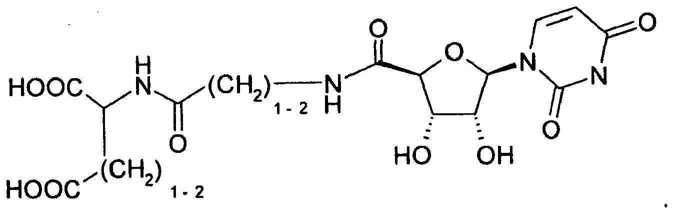

10. The compound of any of claims 1 to 9 which is a selective NTPDase inhibitor.

11. A pharmaceutical or diagnostic composition or a medicament comprising a compound as defined in any one of claims 1 to 9.

12. Use of the compound of any one of claims 1 to 9 for the preparation of a medicament for treating diseases connected with a reduced abundance of nucleotides in a patient or for therapies aiming at increasing the nucleotide concentration in a patient.

13. The use of claim 12, wherein the medicament is for therapy of dry eye disease, respiratory diseases, cystic fibrosis, inflammatory diseases, diseases of the immune system, gastrointestinal diseases, kidney disorders, cancer, and brain diseases.

14. An *in vitro* method for ATP quantification which comprises utilizing the compound of any of claims 1 to 9, preferably in a luciferase assay.

15. A method for preparing the compound of formula (I) of any one of claim 1 to 9, which comprises reacting a compound of formula (II) 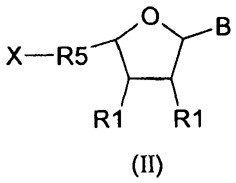 wherein X is a leaving group and all-other variables are as defined in claim 1, with a compound of formula (III) 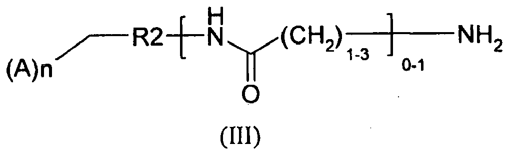 wherein all variables are as defined in claim 1.

## Patentansprüche

1. Verbindung, dargestellt durch die Formel (I) 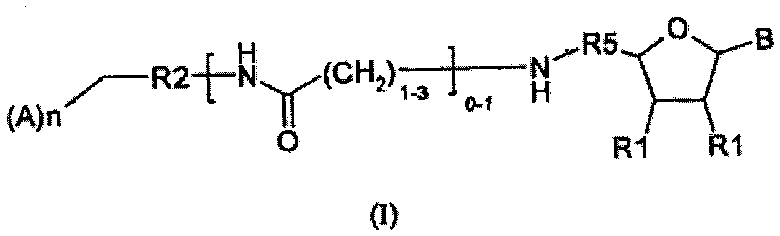 wobei
B für einen Oxopurinyl- oder Oxopyrimidinylrest steht, der über eines seiner Stickstoffatome mit dem Furanosidring verbunden ist;
R1 unabhängig voneinander für Reste stehen, die aus Hydroxy, Wasserstoff, C₁-C₃-Alkoxy, C₁-C₃-Alkyl, C₁-C₃-Alkenyl, C₁-C₃-Alkinyl, C₁-C₃-Acyl, Halogen ausgewählt sind, oder gemeinsam mit einem der vicinalen C-Atome eine Doppelbindung oder miteinander einen Acetyl- oder Ketalring bilden;
R2 -(CH₂)₀₋₂- oder Phenylen ist;
A für einen Rest -PO(OR3)₂ oder -SO₂(OR3) steht, wobei R3 C₁-C₃-Alkyl, Aryl, Arylalkyl oder Heteroaryl ist oder A für einen Rest -(CH₂)ₘ-COOR4 steht, wobei m eine ganze Zahl von 0 bis 2 ist und R4 aus Wasserstoff und C₁-C₃-Alkyl ausgewählt ist;
R5 eine Carbonyl- oder Methylengruppe ist; und
n 1 oder 2 ist, mit der Maßgabe, dass n 2 ist, wenn A für einen Rest -(CH₂)ₘ-COOR4 steht;
oder ein Salz davon.

2. Verbindung gemäß Anspruch 1, die durch die Formel 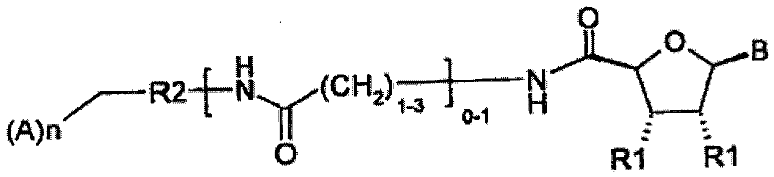 dargestellt wird, wobei B, R1, R2, n und A wie in Anspruch 1 definiert sind.

3. Verbindung gemäß Anspruch 1 oder 2, wobei
(i) wenigstens ein R1 OH ist und das andere R1 H oder OH ist; und/oder
(ii) der Spacer zwischen dem 5'-C des Nucleosids und A wenigstens drei Kohlenstoff- oder Heteroatome umfasst.

4. Verbindung gemäß einem der Ansprüche 1 bis 3, wobei
B aus einem nativen Oxopurinyl- oder Oxopyrimidylrest einschließlich Uracilyl, Cytosinyl, Guaninyl, Hypoxanthinyl und Xanthinyl ausgewählt ist,
wobei B vorzugsweise Uracilyl ist oder am meisten bevorzugt 1-Uracilyl ist.

5. Verbindung gemäß einem der Ansprüche 1 bis 4, wobei A für einen Rest -PO(OR3)₂ steht, R3 Ethyl ist und/oder n 1 ist.

6. Verbindung gemäß Anspruch 5, die durch die Formel 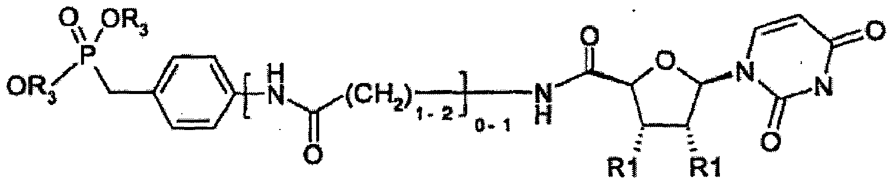 dargestellt wird, wobei vorzugsweise
(i) wenigstens ein R1 OH ist und das andere R1 H oder OH ist, wobei besonders bevorzugt beide R1 OH sind; und/oder
(ii)R3 Ethyl ist.

7. Verbindung gemäß Anspruch 6, die durch die Formel 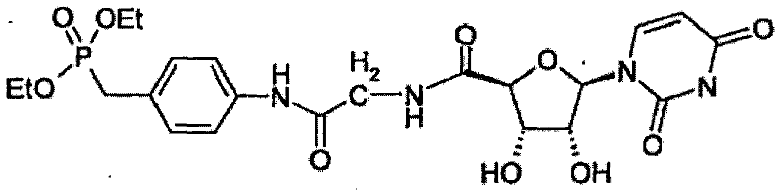 dargestellt wird.

8. Verbindung gemäß einem der Ansprüche 1 bis 4, wobei A für einen Rest -(CH₂)ₘ-COOR4 steht und R4 H ist.

9. Verbindung gemäß Anspruch 8, die durch die Formel 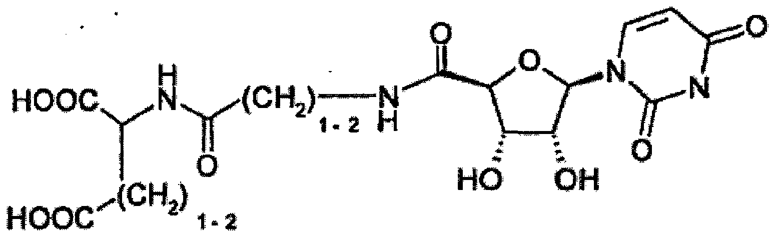 dargestellt wird.

10. Verbindung gemäß einem der Ansprüche 1 bis 9, bei der es sich um einen selektiven NTPDase-Inhibitor handelt.

11. Pharmazeutische oder diagnostische Zusammensetzung oder Medikament, die oder das eine Verbindung gemäß einem der Ansprüche 1 bis 9 umfasst.

12. Verwendung der Verbindung gemäß einem der Ansprüche 1 bis 9 für die Herstellung eines Medikaments zur Behandlung von Erkrankungen, die mit einer reduzierten Häufigkeit von Nucleotiden in einem Patienten verbunden sind, oder für Therapien, die darauf abzielen, die Nucleotidkonzentration in einem Patienten zu erhöhen.

13. Verwendung gemäß Anspruch 12, wobei das Medikament für die Therapie von Keratoconjunctivitis sicca, Atemwegserkrankungen, cystischer Fibrose, Entzündungskrankheiten, Erkrankungen des Immunsystems, Magen-Darm-Erkrankungen, Nierenstörungen, Krebs und Gehirnerkrankungen bestimmt ist.

14. In-vitro-Verfahren für die ATP-Quantifizierung, umfassend die Verwendung der Verbindung gemäß einem der Ansprüche 1 bis 9, vorzugsweise in einem Luciferase-Assay.

15. Verfahren zur Herstellung der Verbindung der Formel (I) gemäß einem der Ansprüche 1 bis 9, umfassend das Umsetzen einer Verbindung der Formel (II) 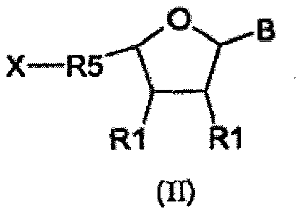 wobei X eine Abgangsgruppe ist und alle anderen Variablen wie in Anspruch 1 definiert sind, mit einer Verbindung der Formel (III) 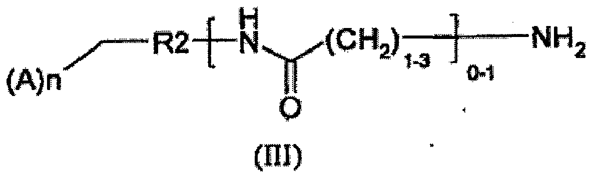 wobei alle Variablen wie in Anspruch 1 definiert sind.

## Revendications

1. Composé représenté par la formule (I) 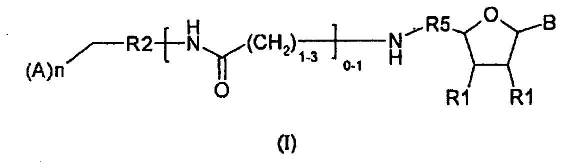 dans lequel
B représente un résidu oxopurinyle ou oxopyrimidinyle qui est relié au cycle furanoside via un de ses atomes d'azote ;
les R1 représentent indépendamment l'un de l'autre des résidus sélectionnés parmi un hydroxyle, l'hydrogène, un alcoxyle en C₁-C₃, un alkyle en C₁-C₃, un alcényle en C₁-C₃, un alcinyle en C₁-C₃, un acyle en C₁-C₃, un halogène, ou forment généralement une double liaison avec un des atomes de C voisins ou un cycle acétyle ou cétal l'un avec l'autre ;
R2 est -(CH₂)₀₋₂- ou phénylène ;
A représente un résidu -PO(OR3)₂ ou -S02(OR3) dans lequel R3 est alkyle en C₁-C₃, aryle, arylalkyle ou hétéroaryle, ou A représente un résidu -(CH₂)ₘ-COOR4, dans lequel m est un nombre entier de 0 à 2 et R4 est sélectionné parmi l'hydrogène et un alkyle en C₁-C₃;
R5 est un groupe carbonyle ou méthylène ; et
n vaut 1 ou 2, à condition que lorsque A représente un résidu -(CH₂)ₘ-COOR4, n valle 2 ;
ou sel de celui-ci.

2. Composé selon la revendication 1, qui est représenté par la formule 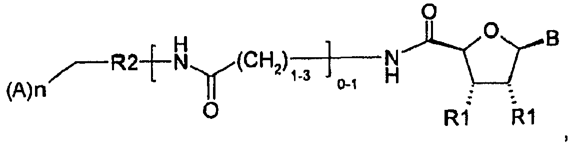 dans lequel B, les R₁, R₂, n et A sont tels qu'ils sont définis dans la revendication 1.

3. Composé selon la revendication 1 ou 2, dans lequel
(i) au moins un R₁ est OH et l'autre R₁ est H ou OH ; et/ou
(ii) l'espaceur entre le nucléoside 5'C et A comprend au mois trois carbones ou hétéroatomes.

4. Composé selon l'une quelconque des revendications 1 à 3, dans lequel
B est sélectionné parmi un résidu oxopurinyle ou oxopyrimidyle natif comprenant l'uracilyle, le cytosinyle, le guaninyle, l'hypoxanthinyle, le xanthinyle,
de préférence B est l'uracilyle ou, le plus préférablement le 1-uracilyle.

5. Composé selon l'une quelconque des revendication 1 à 4, dans lequel A représente un résidu -PO(OR3)₂, R3 est éthyle et/ou n vaut 1.

6. Composé selon la revendication 5, qui est représenté par la formule 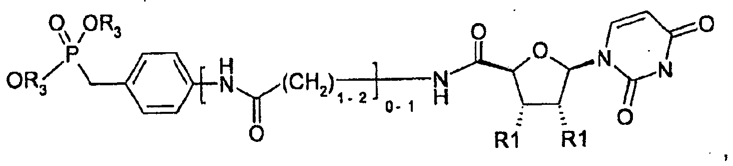 et dans lequel de préférence
(i) au moins un R₁ est OH et l'autre R₁ est H ou OH, plus préférablement les deux R1 sont OH ; et/ou
(ii) R3 est éthyle.

7. Composé selon la revendication 6, qui est représenté par la formule

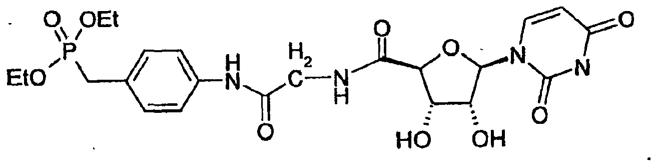

8. Composé selon l'une quelconque des revendications 1 à 4, dans lequel A représente un résidu -(CH₂)ₘ-COOR4 et R4 est H.

9. Composé selon la revendication 8, qui est représenté par la formule

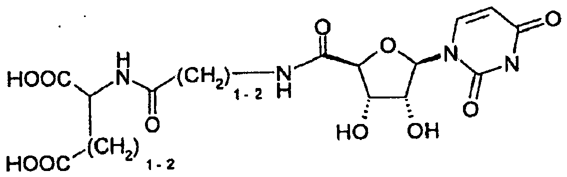

10. Composé selon l'une quelconque des revendications 1 à 9 qui est un inhibiteur sélectif de NTPDase.

11. Composition pharmaceutique ou diagnostique ou médicament comprenant un composé tel qu'il est défini dans l'une quelconque des revendications 1 à 9.

12. Utilisation du composé selon l'une quelconque des revendications 1 à 9 pour la préparation d'un médicament destiné au traitement de maladies liées à une abondance réduite de nucléotides chez un patient ou destiné à des thérapies ayant pour objectif d'augmenter la concentration des nucléotides chez un patient.

13. Utilisation selon la revendication 12, dans lequel le médicament est destiné à une thérapie de la maladie de l'oeil sec, des maladies respiratoires, de la fibrose kystique, des maladies inflammatoires, des maladies du système immunitaire, des maladies gastro-intestinales, des troubles rénaux, du cancer et des maladies cérébrales.

14. Procédé *in vitro* pour la quantification de l'ATP qui comprend l'utilisation du composé selon l'une quelconque des revendications 1 à 9, de préférence dans le dosage de la luciférase.

15. Procédé de préparation du composé de formule (I) selon l'une quelconque des revendications 1 à 9, qui comprend la réaction d'un composé de formule (II) 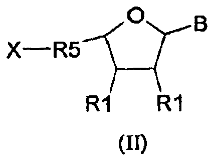 dans lequel X est un groupe partant et toutes les autres variables sont telles que définies dans la revendication 1, avec un composé de formule (III) 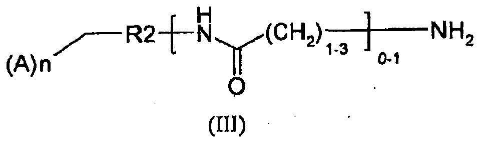 dans lequel toutes les variables sont telles que définies dans la revendication 1.
